(19) **Europäisches Patentamt** **European Patent Office** **Office européen des brevets**

(11) **EP 4 763 974 A1**

(12) **EUROPEAN PATENT APPLICATION**
published in accordance with Art. 153(4) EPC

(43) Date of publication:
24.06.2026 Bulletin 2026/26

(21) Application number: 25861302.5

(22) Date of filing: 27.08.2025

(51) International Patent Classification (IPC):
*C12N 1/38* (2006.01)        *C12N 1/20* (2026.01)
*C12P 13/08* (2006.01)        *C12R 1/15* (2006.01)
*C12R 1/185* (2006.01)

(52) Cooperative Patent Classification (CPC):
**C12N 1/20; C12N 1/38; C12P 13/08;**
C12R 2001/15; C12R 2001/185

(86) International application number:
**PCT/KR2025/013040**

(87) International publication number:
**WO 2026/049489 (05.03.2026 Gazette 2026/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 28.08.2024  KR 20240116276
25.07.2025  KR 20250101523

(71) Applicant: **CJ Cheiljedang Corporation**
**Seoul 04560 (KR)**

(72) Inventors:
• **KWAK, Dong Hun**
**Seoul 04560 (KR)**

• **PARK, Sang Min**
**Seoul 04560 (KR)**
• **PARK, Jeong Ho**
**Seoul 04560 (KR)**
• **LEE, Yong-Chan**
**Seoul 04560 (KR)**
• **LEE, Su Jin**
**Seoul 04560 (KR)**
• **SHIN, Jihyun**
**Seoul 04560 (KR)**

(74) Representative: **Meissner Bolte Partnerschaft
mbB**
**Patentanwälte Rechtsanwälte**
**Postfach 86 06 24**
**81633 München (DE)**

(54) **CULTURE MEDIUM COMPOSITION FOR CORYNEBACTERIUM SP. AND USE THEREOF**

(57)     The present application provides a culture medium composition for a *Corynebacterium* sp. microorganism, in which a portion of ammonium sulfate is replaced with other ammonium-containing compound(s) and a method for producing a basic amino acid using the same.

**EP 4 763 974 A1**

**Description**

[TECHNICAL FIELD]

The present application claims the benefit of the priority based on Korean Patent Application No. 10-2024-0116276 filed on August 28, 2024 and Korean Patent Application No. 10-2025-0101523 filed on July 25, 2025, and the entire contents disclosed in the document of the corresponding Korean patent application are incorporated as a part of the present application.

Throughout the present application, a number of papers and patent documents are cited and citations thereof are indicated. The disclosures of the cited papers and patent documents are incorporated by reference in the present application in their entirety, and the level of the technical field to which the present invention pertains and the contents of the present invention are more clearly described.

The present application provides a culture medium composition for a *Corynebacterium* sp. microorganism, in which a portion of ammonium sulfate is replaced with other ammonium-containing compound(s) and a method for producing a basic amino acid using the same.

[BACKGROUND ART]

Lysine (L-lysine), which is one of basic amino acids is one of essential amino acids, and is used in various fields mainly such as feed additives, food additives, pharmaceutical raw materials, and the like, and is one of bulk chemicals, of which the market size as of 2022 is approximately 2.94 million tons. Currently, most lysine is mass-produced by direct fermentation using a medium containing a carbon source (raw sugar, sucrose, glucose) and a nitrogen source (yeast extract, soybean meal hydrolysate, corn steep liquor).

In ammonium sulfate added among medium components supplied in lysine fermentation using a coryneform microorganism, sulfate serves as a counterion for maintaining a neutral pH condition in relation to lysine biosynthesized during fermentation, and ammonium serves as a nitrogen source required for lysine biosynthesis. As a result, it is present in the form of lysine sulfate in the culture solution, and for production in the form of lysine HCl, which is a powder formulation, a process of adsorption and elution of lysine by an ion exchange resin process is required, and in this process, the complexity of the purification process and addition of auxiliary materials are essential matters, and thus it becomes a factor of significantly increasing manufacturing costs.

In order to overcome the problems, through process simplification by introduction of a granule formulation to a fermented solution (US 8916213 B2), manufacturing costs were significantly reduced, and by advantages of low hygroscopicity and good flowability of the product, through diversification of the formulation, the problems of the process can be overcome and cost competitiveness can be greatly improved. However, since the granulation process conducts granulation without treatment of an ion exchange resin to the fermented solution obtained from the fermentation process, by-products except for lysine, which are generated during fermentation, become a major cause of decreasing the purity of the product generated through the granulation process.

Therefore, it is needed to develop a technology for reducing the amount of input of ammonium sulfate, such as by limiting the amount of input of ammonium sulfate or applying another candidate substitute, or the like, in the fermentation process.

[DISCLOSURE]

[TECHNICAL PROBLEM]

One embodiment of the present application provides culture medium composition for a *Corynebacterium* sp. microorganism, comprising ammonium sulfate and other ammonium-containing compound(s).

Another embodiment provides a method for producing a basic amino acid comprising culturing a *Corynebacterium* sp. microorganism in the culture medium composition.

Other embodiment provides a use of the culture medium composition for producing a basic amino acid.

Other embodiment provides a culture containing a basic amino acid, comprising a *Corynebacterium* sp. microorganism and the culture medium composition.

Other embodiment provides a method for preparing a culture medium for a *Corynebacterium* sp. microorganism, or a method for improving the purity of a basic amino acid of a culture medium for a *Corynebacterium* sp. microorganism, wherein a portion of ammonium sulfate is replaced with other ammonium-containing compound(s), in the culture medium for a *Corynebacterium* sp. microorganism.

[TECHNICAL SOLUTION]

This will be described in detail as follows. Meanwhile, each description and embodiment disclosed in the present description may be applied to each other description and embodiment. In other words, all combinations of various components disclosed in the present application fall within the scope of the present application. In addition, the scope of the present application cannot be seen to be limited by the specific description described below. Furthermore, those skilled in the art can recognize or verify many equivalents to the specific embodiment of the present application described in the present application using only a common experiment. In addition, these equivalents are intended to be included in the present application. Moreover, the numerical values described in the present application may be interpreted to include numerical values in an equivalent or similar range thereof (for example, ±20%, ±15%, ±10%, ±5%, ±3%, or ±1%, etc.).

Hereinafter, the present invention will be described in more detail:

Culture medium composition a *Corynebacterium* sp. microorganism

In one embodiment of the present application, a culture medium composition for a *Corynebacterium* sp. microorganism of the present application,

may comprise ammonium sulfate; and one or more ammonium-containing compounds selected from the group consisting of ammonium succinate, ammonium phosphate, and ammonium acetate, and
one or more values selected from the following [Equation 1] to [Equation 4] may be 2% or more, but not limited thereto:

[(a sum of moles (mol) of ammonium derived from ammonium succinate, ammonium phosphate, and ammonium acetate)/(a sum of moles (mol) of ammonium derived from ammonium sulfate, ammonium succinate, ammonium phosphate, and ammonium acetate)]*100(%)       [Equation 1]

[(a sum of weights (g) of ammonium derived from ammonium succinate, ammonium phosphate, and ammonium acetate)/(a sum of weights (g) of ammonium derived from ammonium sulfate, ammonium succinate, ammonium phosphate, and ammonium acetate)]*100(%)       [Equation 2]

[(a sum of moles (mol) of nitrogen derived from ammonium succinate, ammonium phosphate, and ammonium acetate)/(a sum of moles (mol) of nitrogen derived from ammonium sulfate, ammonium succinate, ammonium phosphate, and ammonium acetate)]*100(%)       [Equation 3]

[(a sum of weights (g) of nitrogen derived from ammonium succinate, ammonium phosphate, and ammonium acetate)/(a sum of weights (g) of nitrogen derived from ammonium sulfate, ammonium succinate, ammonium phosphate, and ammonium acetate)]*100(%).       [Equation 4]

More specifically, the culture medium composition for a *Corynebacterium* sp. microorganism of the present application, may comprise ammonium sulfate; and one or more ammonium-containing compound selected from the group consisting of ammonium succinate, ammonium phosphate, and ammonium acetate, and one or more values selected from [Equation 1] to [Equation 4] described above may be 2% or more to 100% or less, but not limited thereto. More specifically, the one or more values selected from [Equation 1] to [Equation 4] described above may fall within a range formed by one lower limit selected from 2%, 5%, 7%, 10%, 15%, 20%, 25%, 30%, 35%, 40%, 45%, 50%, 55%, 60% and 65% and one upper limit selected from 100%, 99%, 90%, 80%, 70%, 65%, 60%, 55%, 45%, 40%, 35%, 30%, 25%, 20%, 15%, 10% and 5%, wherein the upper limit may be set to be the selected lower limit or more. As one example, the one or more values selected from [Equation 1] to [Equation 4] described above may be in a range of 2% to 100%, 2% to 99%, 2% to 70%, 2% to 50%, 2% to 10%, 5% to 80%, 5% to 70%, 5% to 60%, 5% to 50%, 5% to 35%, 5% to 20%, 5% to 10%, 20% to 30%, 30% to 40%, 40% to 65%, 40% to 50%, 50% to 60%, 60% to 70%, or 65% to 70%, but not limited thereto.
In the present description, the range described as 'x to y,' unless otherwise mentioned, means x or more to y or less.
As described above, the culture medium composition for a *Corynebacterium* sp. microorganism of the present application is characterized by replacing a portion of ammonium sulfate with other ammonium-containing compound(s). In the present application, by replacing ammonium sulfate with one or a combination of two or more ammonium-containing compounds selected from ammonium acetate, ammonium succinate, and ammonium phosphate, which are ammoniac counterion substances, the amount of anionic counterions, specifically, sulfate ions (sulfate), remaining in the fermented solution can be reduced and the purity of the final fermented solution can be improved. Ammonium ions serve as a source of supply of a nitrogen source required for lysine biosynthesis, and acetate and succinate are completely consumed as carbon sources through a microbial metabolic pathway, and phosphate is consumed by nucleic acid and phospholipid biosynthesis of cells, by replacing phosphoric acid added to the conventional medium with ammonium phosphate. Therefore, in the present technology, the purposes are to significantly increase the stability of the fermentation process and to improve the purity of the fermented solution to enhance the product quality, by introducing an ammonium substitute comprising an anionic counterion that can be consumed by a microorganism as a carbon source or phosphorus source (phosphate source), compared to the conventional technology in which the entire amount in the medium is added as ammonium sulfate.

The ammonium sulfate may have a function as a nitrogen source and/or a pH adjusting agent in the culture medium composition, but not limited thereto.

In the present description, the other ammonium-containing compound refers to a substance comprising an anion other than sulfate ion as an anionic counterion of ammonium, and in the present description, it may be interchangeably used with "ammonium sulfate substitute." In one embodiment, in order to reduce the residual amount of the anionic substance in the medium, the other ammonium-containing compound may comprise a substance that can be consumed as a carbon source, phosphorus source, and the like in a microbial metabolic pathway as an anionic counterion of ammonium. For example, the anionic counterion of ammonium may be one or more anions selected from the group consisting of succinate, phosphate, acetate, and the like. In one specific embodiment, the other ammonium-containing compound may comprise one or more selected from the group consisting of ammonium succinate, ammonium phosphate, ammonium acetate, and the like. More specifically, the other ammonium compound may comprise ammonium succinate, ammonium phosphate, or a mixture of ammonium succinate and ammonium phosphate, or further comprise ammonium acetate in addition to ammonium succinate, ammonium phosphate, or a mixture of ammonium succinate and ammonium phosphate. In other embodiment, the other ammonium-containing compound may not comprise one or more selected from the group consisting of urea, ammonium carbonate, ammonium chloride and the like, but not limited thereto.

The culture medium composition for a *Corynebacterium* sp. microorganism provided in the present application can improve production ability (for example, purity, produced amount, etc.) of a target substance (for example, basic amino acid such as L-lysine, L-arginine, and/or L-histidine, etc.), by reducing the residual amount of sulfate ions in the medium after culture and/or fermentation of a microorganism, while the level of AN (ammonium nitrogen) and/or ammonium in the medium is maintained at a comparable level, compared to a case of no replacement, by replacing a portion of ammonium sulfate with other ammonium-containing compound, or comprising ammonium sulfate with other ammonium-containing compound.

In the present application, basic amino acids may refer to amino acids which have one or more basic functional groups (for example, amine group, guanidinium group, imidazole group) on a side chain, in addition to basic amino groups in the molecule, and are positively charged at neutral pH due to this, and can easily form counterions with anions such as sulfate ions ($SO_4^{2-}$). The basic amino acid may be one or more amino acids selected from the group consisting of L-lysine, L-arginine and L-histidine, and more specifically, may be L-lysine, but not limited thereto.

In the culture medium composition of the present application, ammonium sulfate; and one or more ammonium-containing compounds selected from the group consisting of ammonium succinate, ammonium phosphate, and ammonium acetate may be comprised as an ammoniac counterion substance, an ammonium source of supply and/or a pH adjusting agent, but not limited thereto.

In the present application, ammonium sulfate may refer to a compound with the chemical formula of $(NH_4)_2SO_4$ and the molecular weight of 132.14. The ammonium sulfate comprises 2 ammonium ions per 1 molecule, and the counterion is a sulfate ion ($SO_4^{2-}$).

In the present application, ammonium acetate may refer to a compound with the chemical formula of $NH_4CH_3CO_2$, and the molecular weight of 77.0825. The ammonium acetate comprises 1 ammonium ion per 1 molecule, and the counterion is an acetate ion ($CH_3COO^-$).

In the present application, ammonium succinate may refer to a compound with the chemical formula of $(NH_4)_2C_4H_4O_4$, and the molecular weight of 152.15. The ammonium succinate comprises 2 ammonium ions per 1 molecule, and the counterion is a succinate ion ($C_4H_4O_4^{2-}$).

In the present application, ammonium phosphate may refer to a compound with the chemical formula of $(NH_4)_2HPO_4$, and the molecular weight of 132.06. The ammonium phosphate comprises 2 ammonium ions per 1 molecule, and the counterion is a phosphate ion ($HPO_4^{2-}$).

In one embodiment, the culture medium composition for a *Corynebacterium* sp. microorganism of the present application described above, may comprise ammonium sulfate; and one or more ammonium-containing compounds selected from the group consisting of ammonium succinate, ammonium phosphate, and ammonium acetate, and one or more values of following [Equation 5] to [Equation 8] may be 90% or less, but not limited thereto:

[(moles (mol) of ammonium derived from ammonium acetate)/(a sum of moles (mol) of ammonium derived from ammonium succinate, ammonium phosphate, and ammonium acetate)]*100(%).  [Equation 5]

[(weight (g) of ammonium derived from ammonium acetate)/(sum of weights (g) of ammonium derived from ammonium succinate, ammonium phosphate, and ammonium acetate)]*100(%)  [Equation 6]

[(moles (mol) of nitrogen derived from ammonium acetate)/(a sum of mol of ammonium derived from ammonium succinate, ammonium phosphate, and ammonium acetate)]*100(%)  [Equation 7]

[(weight (g) of nitrogen derived from ammonium acetate)/(a sum of weights (g) of nitrogen derived from ammonium succinate, ammonium phosphate, and ammonium acetate)]*100(%).  [Equation 8]

More specifically, the culture medium composition for a *Corynebacterium* sp. microorganism of the present application described above, may comprise ammonium sulfate; and one or more ammonium-containing compounds selected from the group consisting of ammonium succinate, ammonium phosphate, and ammonium acetate, and one or more values selected from [Equation 5] to [Equation 8] described above may be 0% or more to 90% or less, but not limited thereto. More specifically, the one or more values selected from [Equation 5] to [Equation 8] described above may fall within a range formed by one lower limit selected from 0%, 2%, 5%, 10%, 20%, 30%, 34%, 40%, 50%, and 60%, and one upper limit selected from 90%, 75%, 70%, 67%, 60%, 50%, 40%, 45%, 34%, 30%, 20%, 10%, 5%, 2%, and 1% or less, and the upper limit may be set to the selected lower limit or more. As one example, the one or more values selected from [Equation 5] to [Equation 8] described above may be within a range of 0% or more to 75% or less, 0% or more to 70% or less, 0% or more to 67% or less, 0% or more to 45% or less, 0% or more to 34% or less, 0% or more to 10% or less, or 0% or more to 1% or less, but not limited thereto.

In such an embodiment, the case of 0% refers to that ammonium acetate is not comprised (i.e., absence of ammonium acetate, or comprising ammonium acetate of 0 mol or 0g).

In one specific embodiment, the culture medium composition for a *Corynebacterium* sp. microorganism of the present application described above may comprise ammonium succinate and/or ammonium phosphate as the 'other ammonium-containing compound,' and not comprise ammonium acetate (comprising ammonium acetate of 0 mol or 0g, one or more values selected from [Equation 5] to [Equation 8] described above is 0%). In another specific embodiment, the culture medium composition for a *Corynebacterium* sp. microorganism of the present application described above may further comprise ammonium acetate in addition to ammonium succinate and/or ammonium phosphate. When the culture medium composition for a *Corynebacterium* sp. microorganism of the present application described above further comprises ammonium acetate in addition to ammonium succinate and/or ammonium phosphate, ammonium acetate may be comprised so that one or more values selected from [Equation 5] to [Equation 8] described above is more than 0% and 90% or less, more than 0% and 75% or less, more than 0 and 70% or less, more than 0% and 67% or less, more than 0% and 60% or less, more than 0% and 50% or less, more than 0% and 45% or less, more than 0% and 40% or less, more than 0% and 34% or less, more than 0% and 30% or less, more than 0% and 20% or less, more than 0% and 10% or less, more than 0% and 5% or less, more than 0% and 2% or less, or more than 0% and 1% or less, but not limited thereto.

In one embodiment, the sum of moles (mol) of ammonium derived from ammonium sulfate, ammonium succinate, ammonium phosphate, and ammonium acetate described above may be 0.1 mol to 0.5 mol per 1L of the culture medium composition, but not limited thereto. More specifically, the sum of moles (mol) of ammonium derived from ammonium sulfate, ammonium succinate, ammonium phosphate, and ammonium acetate may be 0.1 mol to 0.5 mol, 0.1 mol to 0.47 mol, 0.1 mol to 0.465 mol, 0.1 mol to 0.46 mol, 0.1 mol to 0.4 mol, 0.1 mol to 0.3 mol, 0.1 mol to 0.275 mol, 0.1 mol to 0.265 mol, 0.1 mol to 0.26 mol, 0.1 mol to 0.225 mol, 0.1 mol to 0.2 mol, 0.15 mol to 0.5 mol, 0.15 mol to 0.47 mol, 0.15 mol to 0.465 mol, 0.15 mol to 0.46 mol, 0.15 mol to 0.4 mol, 0.15 mol to 0.3 mol, 0.15 mol to 0.275 mol, 0.15 mol to 0.265 mol, 0.15 mol to 0.26 mol, 0.15 mol to 0.225 mol, 0.15 mol to 0.2 mol, 0.18 mol to 0.5 mol, 0.18 mol to 0.47 mol, 0.18 mol to 0.465 mol, 0.18 mol to 0.46 mol, 0.18 mol to 0.4 mol, 0.18 mol to 0.3 mol, 0.18 mol to 0.275 mol, 0.18 mol to 0.265 mol, 0.18 mol to 0.26 mol, 0.18 mol to 0.225 mol, or 0.18 mol to 0.2 mol per 1L of the culture medium composition, but not limited thereto.

In one embodiment, the culture medium composition of the present application described above, may comprise one or more selected from the group consisting of

0.05 mol to 1.2 mol of ammonium derived from ammonium succinate,
0.01 mol to 0.5 mol of ammonium derived from ammonium phosphate, and

0.05 mol to 1 mol of ammonium derived from ammonium acetate,
based on 1 mol of ammonium derived from ammonium sulfate, but not limited thereto.

In one specific embodiment, the culture medium composition of the present application described above, may comprise one or more selected from the group consisting of

0.05 mol to 1.2 mol of ammonium derived from ammonium succinate, more specifically, ammonium succinate in an amount within a range formed by one lower limit selected from 0.05 mol, 0.1 mol, 0.115 mol, 0.17 mol, 0.275 mol, 0.35 mol, 0.4 mol and 0.8 mol, and one upper limit selected from 1.2 mol, 1.175 mol, 1 mol, 0.8 mol, 0.4 mol, 0.35 mol, 0.275 mol, 0.17 mol and 0.0115 mol (wherein the upper limit may be set to the selected lower limit or more),
0.01 mol to 0.5 mol of ammonium derived from ammonium phosphate, more specifically, ammonium phosphate in an amount within a range formed by one lower limit selected from 0.01 mol, 0.05 mol, 0.07 mol, 0.09 mol, 0.15 mol, 0.2 mol and 0.3 mol, and one upper limit selected from 0.5 mol, 0.4 mol, 0.3 mol, 0.2 mol, 0.15 mol and 0.09 mol (wherein the upper limit may be set to the selected lower limit or more), and
0.05 mol to 1 mol of ammonium derived from ammonium acetate, more specifically, ammonium acetate in an amount within a range formed by one lower limit selected from 0.05 mol, 0.09 mol, 0.165 mol, 0.275 mol, 0.4 mol and 0.5 mol, and one upper limit selected from 1 mol, 0.9 mol, 0.5 mol, 0.4 mol, 0.275 mol and 0.165 mol (wherein the upper limit may be set to the selected lower limit or more),
based on 1 mol of ammonium derived from ammonium sulfate, but not limited thereto.

In the present application, the term "medium" refers to a material in which nutrients required for culturing a microorganism are mixed as main components, and supplies nutrients and growth factors and the like including water essential for survival and growth. Any medium that meets general culture conditions of a microorganism selected for production of a target substance (for example, basic amino acid such as L-lysine, L-arginine, and/or L-histidine, etc.) may be used without particular limitation, and may be one which contains appropriate carbon sources, nitrogen sources, phosphorus sources, inorganic compounds, amino acids, and/or vitamins, and the like depending on the selected microorganism, and in which temperature, pH and the like are adjusted under aerobic conditions.

The carbon source may comprise one or more selected from the group consisting of carbohydrates (sugars) such as glucose, lactose, fructose, sucrose, maltose, etc.; sugar alcohols such as mannitol, sorbitol, etc., organic acids such as pyruvate, lactate, citrate, and the like; amino acids such as glutamate, methionine, lysine, etc., and the like. In addition, one or more natural organic nutrients selected from the group consisting of starch hydrolysates, molasses, black strap molasses, rice bran, cassava, sugar cane molasses, corn steep liquor, and the like may be used. For example, as the carbon source, carbohydrates such as corn steep liquor, glucose, sterilized pre-treated molasses (i.e., molasses converted into reducing sugar) and the like may be used, and in addition, carbon sources of appropriate type and proper amount may be variously used without limitation. These carbon sources may be used alone or used in combination of two or more kinds.

In one embodiment, the culture medium composition of the present application described above may comprise the carbon source (for example, sugar) in an amount of 10 g to 100 g per 1L of the culture medium composition, and more specifically, in an amount of 10 g to 100 g, 10 g to 70 g, 10 g to 60 g, 10 g to 50 g, 10 g to 40 g, 20 g to 100 g, 20 g to 70 g, 20 g to 60 g, 20 g to 50 g, or 20 g to 40 g per 1L of the culture medium composition, but not limited thereto.

As the nitrogen source, one or more selected from the group consisting of inorganic nitrogen sources such as ammonia, ammonium chloride, ammonium carbonate, ammonium nitrate, etc.; organic nitrogen sources such as amino acids like glutamate, methionine, glutamine, etc., peptone, NZ-amine, meat extract, yeast extract, malt extract, corn steep liquor, casein hydrolysate, fish or decomposition products thereof, defatted soybean cake or decomposition products thereof, etc., and the like may be used, but not limited thereto. These nitrogen sources may be used alone or used in combination of two or more kinds.

The phosphorus source may include one or more selected from the group consisting of phosphate, potassium phosphate monobasic, potassium phosphate dibasic, or sodium-containing salts corresponding thereto, and the like. When the culture medium composition of the present application comprises ammonium phosphate as a substitute of ammonium sulfate, the content of the phosphorus source may be adjusted depending on the content of the ammonium phosphate. For example, the culture medium composition of the present application may comprise the phosphorus source in an amount equal to the amount obtained by subtracting the amount of phosphorus comprised in the ammonium phosphate from the total amount of phosphorus required for culture of a microorganism producing a target substance and/or production of a target substance, or may not comprise a separate phosphorus source substance, when the content of the ammonium phosphate is sufficient.

As the inorganic compound, sodium chloride, calcium chloride, iron chloride, magnesium sulfate, iron sulfate, manganese sulfate, calcium carbonate, and the like may be used, and in addition, amino acids, vitamins, and/or appropriate precursors, and the like may be included.

In the present application, the culture medium composition of the present application described above may have a pH of 6 to 9. More specifically, the culture medium composition of the present application may have a pH of 6 to 9, 6 to 8.75, 6 to 8.5, 6.25 to 9, 6.25 to 8.75, 6.25 to 8.5, 6.5 to 9, 6.5 to 8.75, or 6.5 to 8.5, but not limited thereto. In one embodiment, in order to maintain the pH range of the culture medium composition in the culturing step, ammonia gas and/or ammonia solution may be added to the culture medium composition. By maintaining the pH range as above, a metabolic environment advantageous for growth and development of a *Corynebacterium* sp. microorganism and production of target products (for example, basic amino acids such as L-lysine, L-arginine, and/or L-histidine) may be provided.

The component or precursor described above may be added to the medium in a batch or continuous manner, but not limited thereto.

In the present application, the medium may be a liquid, solid medium, or a semisolid medium, and in one embodiment, it may be a liquid medium, but not limited thereto.

In one embodiment, the culture medium composition for a *Corynebacterium* sp. microorganism provided in the present application may be used as a seed culture medium, a main culture medium, or both. Accordingly, the culture medium composition for a *Corynebacterium* sp. microorganism of the present application may be a seed culture medium composition or main culture medium composition. In one specific embodiment, the culture medium composition for a *Corynebacterium* sp. microorganism of the present application may be a seed culture medium composition. In another specific embodiment, the culture medium composition for a *Corynebacterium* sp. microorganism of the present application may be a main culture medium composition. In other specific embodiment, the culture medium composition for a *Corynebacterium* sp. microorganism of the present application may be used in both the seed culturing step and the main culturing step, respectively, and in such an embodiment, the composition applied to each step may independently configured differently from each other, while satisfying the conditions (or characteristics) described in the present description.

In one specific embodiment, the seed culture medium composition of the present application may not comprise ammonium acetate (comprising 0 mol or 0 g), or may further comprise ammonium acetate in addition to ammonium succinate and/or ammonium phosphate.

More specifically, the seed culture medium composition of the present application may correspond to one or more of the following:

a seed culture medium composition comprising ammonium sulfate, and ammonium succinate,
wherein the amount of ammonium derived from the ammonium succinate is 0.05 mol to 1.2 mol,
based on 1 mol of ammonium derived from the ammonium sulfate;
a seed culture medium composition comprising ammonium sulfate, and ammonium phosphate,
wherein the amount of ammonium derived from the ammonium phosphate is 0.01 mol to 0.5 mol,
based on 1 mol of ammonium derived from the ammonium sulfate;
a seed culture medium composition comprising ammonium sulfate, ammonium succinate and ammonium phosphate,
wherein the amount of ammonium derived from the ammonium succinate is 0.05 mol to 1.2 mol, and
the amount of ammonium derived from the ammonium phosphate is 0.01 mol to 0.5 mol,
based on 1 mol of ammonium derived from the ammonium sulfate;
a seed culture medium composition comprising ammonium sulfate, ammonium succinate, and ammonium acetate,
wherein the amount of ammonium derived from the ammonium succinate is 0.05 mol to 1.2 mol, and
the amount of ammonium derived from the ammonium acetate is 0.05 mol to 1 mol,
based on 1 mol of ammonium derived from the ammonium sulfate;
a seed culture medium composition comprising ammonium sulfate, ammonium phosphate, and ammonium acetate,
wherein the amount of ammonium derived from ammonium phosphate is 0.01 mol to 0.5 mol, and
the amount of ammonium derived from ammonium acetate is 0.05 mol to 1 mol,
based on 1 mol of ammonium derived from the ammonium sulfate; and
a seed culture medium composition comprising ammonium sulfate, ammonium succinate, ammonium phosphate and ammonium acetate,
wherein the amount of ammonium derived from ammonium succinate is 0.05 mol to 1.2 mol, and
the amount of ammonium derived from ammonium phosphate is 0.01 mol to 0.5 mol, and
the amount of ammonium derived from ammonium acetate is 0.05 mol to 1 mol,
based on 1 mol of ammonium derived from the ammonium sulfate.

In such an embodiment, the contents (mol) and range of the ammonium derived from ammonium succinate, ammonium derived from ammonium phosphate, and ammonium derived from ammonium acetate based on 1 mol of ammonium derived from ammonium sulfate are as described above.

In the present application, the seed culture medium may refer to a medium for proliferating microorganisms to secure a sufficient number of microorganisms for production of target substances (for example, basic amino acids such as L-lysine, L-arginine, and/or L-histidine). The main culture medium may refer to a medium for producing target substances (for example, basic amino acids such as L-lysine, L-arginine, and/or L-histidine). The culture medium composition of the present application may be used as a seed culture medium, a main culture medium, or both. In addition, the 'other ammonium-containing compound' described above may be comprised in the seed culture medium, main culture medium, or both. The components of the seed culture medium and main culture medium may be same as or different from each other, and specific components are as described above.

In one embodiment, the culture medium composition of the present application may have a reduced sulfate content at the end of culturing, compared to cases of using a culture medium that comprise none of ammonium-containing compound selected from the group consisting of ammonium succinate, ammonium phosphate, and ammonium acetate, more specifically, a culture medium comprising ammonium derived from the 'other ammonium-containing compound' in an amount of less than 0.05 mol, based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5% or less than 7% (including a case of comprising none of 'other ammonium-containing compound' described above).

In one embodiment, the culture medium composition of the present application may be a seed culture medium composition, and the seed culture medium composition may have a reduced sulfate concentration (g/L) in the medium by 10% or more, for example, 10 to 50%, 10 to 40%, 10% to 35%, 10 to 30%, or 10 to 20%, while maintaining the cell density and specific growth rate at the end of seed culture (e.g., at the time of depletion of residual sugars in the medium) at a comparable level, by comprising the 'other ammonium-containing compound' described above, compared to a culture medium comprising ammonium derived from the 'other ammonium-containing compound' in an amount of less than 0.05 mol based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including a case of comprising none of 'other ammonium-containing compound' described above) (See Example 1 and Table 1).

In addition, the present application may be a seed culture medium composition and/or a main culture medium composition, and the seed culture medium composition and/or main culture medium composition may have a sulfate concentration (g/L) in the medium reduced by 5% or more, for example, 5 to 60%, 5 to 55%, 5 to 50%, 5 to 55%, or 5 to 40%, at the end of the main culture, and/or have purity of basic amino acids (for example, L-lysine, L-arginine, and/or L-histidine) in the medium increased by about 1% or more, about 1.5% or more, about 2% or more, about 3% or more, about 5% or more, (the upper limit is not particularly limited, and for example, it may be about 30% or less, about 20% or less, about 10% or less, about 8% or less, or about 7% or less) at the end of main culture, by comprising the 'other ammonium-containing compound' described above, compared to a culture medium which comprise none of ammonium-containing compound selected from the group consisting of ammonium succinate, ammonium phosphate, and ammonium acetate, more specifically, a culture medium comprising ammonium derived from the 'other ammonium-containing compound' in an amount of less than 0.05 mol based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including a case of comprising none of the 'other ammonium-containing compound' described above) (See Example 2 and Table 2).

In the present application, the *Corynebacterium* sp. (or the genus Corynebacterium) microorganism may be a microorganism which produces or can produce a target substance (for example, basic amino acids such as L-lysine, L-arginine, and/or L-histidine, and the like) by fermenting a substrate in a medium, and may include not only a microorganism which originally has production ability of a target substance (wild type), but also a microorganism modified to have production ability of a target substance, and/or a microorganism modified to enhance production ability of a target substance.

In one embodiment, the *Corynebacterium* sp. microorganism may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* and more specifically, it may be *Corynebacterium glutamicum* or *Corynebacterium stationis,* but not limited thereto.

In one specific embodiment, the *Corynebacterium* sp. microorganism may be *Corynebacterium glutamicum* ATCC13032, *Corynebacterium glutamicum* ATCC14067, *Corynebacterium glutamicum* ATCC13869, or a strain mutated to produce a target product using them as a parent strain (for example, *Corynebacterium glutamicum* CJ3P (US 9556463 B2), *Corynebacterium glutamicum* KCCM11016P), but not limited thereto.

In one embodiment, the culture medium composition for a *Corynebacterium* sp. microorganism described above may be a culture medium composition which can be used for culturing a *Corynebacterium* sp. microorganism and/or producing a basic amino acid by the *Corynebacterium* sp. microorganism. The culture medium composition may be used as a main culture medium composition for main culture that produces basic amino acids which are target substances, a seed culture medium composition for seed culture (achieving sufficient proliferation of microorganisms or sufficient cell density for producing a basic amino acid or securing a sufficient number of microorganisms), or both. Accordingly, the culture medium composition of the present application may be a seed culture medium composition for producing a basic amino acid or a main culture medium composition for producing a basic amino acid.

Method for producing a basic amino acid

Other embodiment of the present application, provides a method for producing a basic amino acid, comprising culturing a *Corynebacterium* sp. microorganism in the culture medium composition for a *Corynebacterium* sp. microorganism described above.

More specifically, the method for producing a basic amino acid may comprise

preparing the culture medium composition of the present application described above by adding ammonium sulfate, and other ammonium-containing compound(s) (for example, one or more selected from the group consisting of ammonium succinate, ammonium phosphate, and ammonium acetate); and
culturing a *Corynebacterium* sp. microorganism in the culture medium composition.

The details regarding the culture medium composition for a *Corynebacterium* sp. microorganism (seed culture medium composition, main culture medium composition), the basic amino acids, the other ammonium-containing compound and the like are as described above.

In the method for producing a basic amino acid of the present application, "culturing" may be performed either as a single culturing step in which both proliferation of the *Corynebacterium* sp. microorganism and production of a basic amino acid occur, or may be divided into a seed culture for proliferation of a *Corynebacterium* sp. microorganism and a main culture for production of a basic amino acid.

In one embodiment, when the culturing is performed as a single culturing step, the method for producing a basic amino acid may comprise culturing a *Corynebacterium* sp. microorganism in the seed culture medium composition or main culture medium composition described above.

In another embodiment, the culturing may comprise a seed culture step for proliferating a *Corynebacterium* sp. microorganism and a main culture step for producing a basic amino acid. In such an embodiment, the method for producing a basic amino acid, may comprise a seed culture step of culturing a *Corynebacterium* sp. microorganism in a seed culture medium, and a main culture step of culturing the microorganism cultured in the seed culture step in a main culture medium.

In the method for producing a basic amino acid, the culturing (seed culture or main culture) may be interpreted to comprise inoculating the microorganism into a culture medium composition (seed culture medium composition or main culture medium composition) and culturing the inoculated microorganism under appropriate conditions for proliferation of the microorganism (seed culture) or production of basic amino acids (main culture).

The method for producing a basic amino acid may further comprise recovering, isolating, or purifying basic amino acids from the culture obtained in the culturing, after the culturing (seed culture and/or main culture).

The specific composition of the culture medium composition for a *Corynebacterium* sp. microorganism (seed culture medium composition, main culture medium composition) described above is as described above.

In one embodiment, the method for producing a basic amino acid, may further comprise adding 'other ammonium-containing compound' described above to the seed culture medium composition and/or main culture medium composition. In such an embodiment, the type and added amount of the 'other ammonium-containing compound' are as described above.

In the present application, the term "culture" refers to proliferating a microorganism under appropriately controlled environmental conditions. The culturing process of the present application may be performed under suitable medium and culture conditions known in the art. These culture conditions may be appropriately adjusted by those skilled in the art depending on the microorganism to be cultured. Specifically, the culture may be performed in a batch, continuous, and/or fed-batch, but not limited thereto.

The method of the culture is not particularly limited, and for example, it may be performed by batch culture, fed-batch culture, continuous culture, or a combination of two or more of them, but not limited thereto.

When the culture is performed separately as seed culture and main culture, the seed culture and main culture may be performed by the same or different method from each other, and for example, it may be performed by an independently selected method among the culture method described above, respectively. In one specific embodiment, the seed culture may be performed by batch culture, and the main culture may be performed by fed-batch culture or continuous culture, but not limited thereto.

In addition, when the culture is separately as seed culture and main culture, the seed culture may be performed by using the seed culture medium composition of the present application described above, and the main culture may be performed by using the main culture medium composition of the present application described above. The details regarding the ammonium sulfate, and ammonium succinate, ammonium phosphate, and ammonium acetate, contents thereof, components, production of basic amino acids, increase in production (produced amount) of basic amino acids, increase in purity of basic amino acids, and the like of the seed culture medium composition and main culture medium composition of the present application are as described in other embodiments.

The seed culture may refer to culture of which main purpose is to proliferate microorganisms until the initial microbial concentration for main culture is sufficiently reached. In one specific embodiment, the seed culture may be for example, batch culture, and may be performed under the conditions of pH 6~9 or pH 6.5-8.5, agitation speed of 350 to 550 rpm, 400 to 500rpm, or about 450 rpm, aeration rate of 0.1 to 2 vvm, 0.5 to 1.5 vvm, or about 1 vvm until initial carbon sources of the medium are depleted, but not limited thereto. The pH may be adjusted in the above range by adding ammonia gas, but not limited thereto. The seed culture may refer to inoculating microorganisms into the seed culture medium composition described above, and culturing them under the conditions described above.

The main culture may refer to a process of culturing microorganisms proliferated in the seed culture under conditions suitable for production of target substances (for example, basic amino acids such as L-lysine, L-arginine, and/or L-histidine, etc.) so that production of the target substances occurs primarily form the microorganisms (may be interchangeably used with "fermentation"), and during this process, microbial proliferation may not occur or occur less. In one specific embodiment, the main culture may be for example, fed-batch culture, and may be performed under conditions of pH 6-9 or pH 6.5-8.5, agitation speed of 350 to 550 rpm, 400 to 500rpm, or about 450 rpm, aeration rate of 0.1 to 2 vvm, 0.5 to 1.5 vvm, or about 1 vvm, and the pH may be adjusted in the above range by adding ammonia gas, but not limited thereto. In this case, a feeding medium may be further introduced to the main culture medium as the culture progresses. More specifically, when the initial carbon source in the medium is depleted, a carbon source (e.g., glucose, etc.) is additionally introduced to maintain the residual sugar level in the medium at about 0.1 to 2 wt%, 0.1 to 1 wt%, 0.5 to 2 wt%, or 0.5 to 1 wt%, and when the nitrogen (AN: ammonium nitrogen) content in the medium is less than about 2 g/kg, less than about 1.5 g/kg, less than about 1 g/kg, or less than about 0.5 g/kg, ammonia gas may be introduced to supply the nitrogen source while performing the culture, but is not limited thereto. In the present application, during the culturing, the pH of the culture medium composition may be maintained at 6 to 9. More specifically, the pH of the culture medium composition may be maintained at 6 to 9, 6 to 8.75, 6 to 8.5, 6.25 to 9, 6.25 to 8.75, 6.25 to 8.5, 6.5 to 9, 6.5 to 8.75, or 6.5 to 8.5, but not limited thereto. In one embodiment, in the culturing, in order to maintain the pH range of the culture medium composition, ammonia gas and/or ammonia solution may be added to the culture medium composition. By maintaining the pH range as above, metabolic environments advantageous for growth of *Corynebacterium* sp. microorganisms and production of target products (for example, basic amino acids such as L-lysine, L-arginine, and/or L-histidine, etc.) can be provided.

In one embodiment, in the method for producing a basic amino acid comprising culturing *Corynebacterium* sp. microorganisms in the culture medium composition of the present application described above, the culture medium composition may be a seed culture medium composition.

In one specific embodiment, the method for producing a basic amino acid may further comprise culturing the *Corynebacterium* sp. microorganism by introducing the seed culture medium composition comprising the *Corynebacterium* sp. microorganism into a main culture medium.

In one specific embodiment, the seed culture medium composition comprising the *Corynebacterium* sp. microorganism may be introduced in an amount of 0.1L to 0.4L per 1 L of the main culture medium, and more specifically, it may be introduced in an amount of 0.1L to 0.4L, 0.1L to 0.3L, 0.1L to 0.25L, 0.1L to 0.225L, 0.1L to 0.2L, 0.15L to 0.4L, 0.15L to 0.3L, 0.15L to 0.25L, 0.15L to 0.225L, 0.15L to 0.2L, 0.175L to 0.4L, 0.175L to 0.3L, 0.175L to 0.25L, 0.175L to 0.225L, 0.175L to 0.2L, 0.2L to 0.4L, 0.2L to 0.3L, 0.2L to 0.25L, or 0.2L to 0.225L, per 1 L of the main culture medium, but not limited thereto.

In one specific embodiment, a feeding medium may be further introduced during culturing the *Corynebacterium* sp. microorganism by introducing the seed culture medium composition comprising the *Corynebacterium* sp. microorganism into a main culture medium, but not limited thereto. More specifically, when initial carbon sources in the medium are depleted as the main culture progresses, carbon sources (for example, glucose, etc.) may be further introduced to maintain the residual sugar level in the medium at about 0.1 to 2wt%, 0.1 to 1wt%, 0.5 to 2wt%, or 0.5 to 1wt%, but not limited thereto.

Production of basic amino acids by the method for producing described above, may be confirmed by a known method commonly used for detection and/or identification of compounds (for example, L-lysine, L-arginine, and/or L-histidine). This method may include one or more selected from the group consisting of HPLC, LC/MS, GC/MS, NMR, and the like as an example, but not limited thereto.

The recovery of basic amino acids in the culture (fermented product; may include cells or cell lysates and/or media in which culture is completed) may be performed by a known method commonly used for isolation and/or purification of compounds (for example, L-lysine, L-arginine, and/or L-histidine). Examples of this method, may include one or more selected from the group consisting of, for example, precipitation, membrane separation, filtration (for example, ultrafiltration), centrifugation, dialysis, salting out, various kinds of chromatography such as gel filtration chromatography, adsorption chromatography, ion exchange chromatography, affinity chromatography, etc. and the like, but not limited thereto. When basic amino acids are accumulated in microbial cells, basic amino acids may be recovered, for example, from supernatant obtained by lysing microbial cells by ultrasonication and the like, and removing the microbial cells by centrifugation by an ion exchange resin method and the like, but not limited thereto.

The recovered basic amino acids may be free amino acids and/or salts thereof. The salts may include, for example, sulfate, hydrochloride, carbonate, ammonium salts, sodium salts, potassium salts, but not limited thereto.

The method for producing a basic amino acid of the present application, may additionally comprise a purification step. The purification may be performed, by using an appropriate method known in the art. In one embodiment, when the method for producing a basic amino acid of the present application comprises both a recovery step and a purification step, the recovery step and purification step may be continuously or non-continuously performed in any order, or may be performed simultaneously or by being integrated into one step, but not limited thereto.

In one embodiment of the method for producing a basic amino acid of the present application, the sulfate content at the end of the culturing may be reduced compared to a case of using a culture medium that comprises none of ammonium-containing compound selected from the group consisting of ammonium succinate, ammonium phosphate, and ammonium acetate. Specifically, the method for producing a basic amino acid of the present application may have a reduced sulfate content at the end of culture, by using the culture medium composition of the present application described above, that is, by using the culture medium composition comprising the 'other ammonium-containing compound' described above, compared to cases of using a culture medium that comprise none of ammonium-containing compound selected from the group consisting of ammonium succinate, ammonium phosphate, and ammonium acetate, more specifically, a culture medium comprising ammonium derived from the 'other ammonium-containing compound' in an amount of less than 0.05 mol, based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including a case of comprising none of 'other ammonium-containing compound' described above).

More specifically, the method for producing a basic amino acid of the present application may have a reduced sulfate concentration (g/L) in the medium by 10% or more, for example, 10 to 50%, 10 to 40%, 10% to 35%, 10 to 30%, or 10% to 20%, while maintaining the cell density and specific growth rate at the end of seed culture (e.g., at the time of depletion of residual sugars or at the time when the residual concentration in the medium is about 1%(w/v) or less) at a comparable level, by using the seed culture medium composition of the present application described above, that is, by using the seed culture medium composition comprising the 'other ammonium-containing compound' described above in the seed culture step, compared to cases of using a culture medium which comprise none of ammonium-containing compound selected from the group consisting of ammonium succinate, ammonium phosphate, and ammonium acetate, more specifically, a culture medium comprising ammonium derived from the 'other ammonium-containing compound' in an amount of less than 0.05 mol based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including a case of comprising none of 'other ammonium-containing compound' described above) (See Example 1 and Table 1).

In addition, the method for producing a basic amino acid of the present application may have a sulfate concentration (g/L) in the medium reduced by 5% or more, for example, 5 to 60%, 5 to 55%, 5 to 50%, 5 to 55%, or 5 to 40%, at the end of main culture, by using the seed culture medium composition of the present application described above and/or the main culture medium composition of the present application described above, that is, the seed culture medium composition and/or main culture medium composition that comprise the 'other ammonium-containing compound' described above, in the seed culture step and/or main culture step, compared to cases of using a culture medium which comprise none of ammonium-containing compound selected from the group consisting of ammonium succinate, ammonium phosphate, and ammonium acetate, more specifically, a culture medium comprising ammonium derived from the 'other ammonium-containing compound' in an amount of less than 0.05 mol based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including a case of comprising none of 'other ammonium-containing compound' described above) (See Example 2 and Table 2).

In one embodiment of the method for producing a basic amino acid of the present application, the purity of the basic amino acid at the end of the culturing may be increased, compared to a case of using a culture medium that comprises none of ammonium-containing compound selected from the group consisting of ammonium succinate, ammonium phosphate, and ammonium acetate. Specifically, the method for producing a basic amino acid of the present application may have increased purity of basic amino acids (for example, L-lysine, L-arginine, and/or L-histidine) at the end of culture, by using the culture medium composition of the present application described above, that is, by using the culture medium composition comprising the 'other ammonium-containing compound' described above, compared to cases of using a culture medium which comprise none of ammonium-containing compound selected from the group consisting of ammonium succinate, ammonium phosphate, and ammonium acetate, a culture medium comprising ammonium derived from 'other ammonium-containing compound' in an amount of less than 0.05 mol based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including a case of comprising none of 'other ammonium-containing compound' described above).

More specifically, the method for producing a basic amino acid of the present application may have purity of basic amino acids (for example, L-lysine, L-arginine, and/or L-histidine) in the medium increased by about 1% or more, about 1.5% or more, about 2% or more, about 3% or more, about 5% or more (the upper limit is not particularly limited, and may be about 30% or less, about 20% or less, about 10% or less, about 8% or less, or about 7% or less) at the end of main culture, by using the seed culture medium composition of the present application described above and/or the main culture medium composition of the present application described above, that is, by using the seed culture medium composition and/or main culture medium comprising the 'other ammonium-containing compound' described above, in the seed culture step and/or main culture step, compared to cases of using a culture medium which comprise none of ammonium-containing compound selected from the group consisting of ammonium succinate, ammonium phosphate, and ammonium acetate, more specifically, a culture medium comprising ammonium derived from the 'other ammonium-containing compound' in an amount of less than 0.05 mol based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including a case of comprising none of 'other ammonium-containing compound' described above) (See Example 2 and Table 2).

Culture containing a basic amino acid

Other embodiment provides a culture containing a basic amino acid, comprising a *Corynebacterium* sp. microorganism and the culture medium composition for a *Corynebacterium* sp. microorganism of the present application described above.

The details regarding *Corynebacterium* sp. microorganism and the basic amino acid are as described above.

The culture medium composition for a *Corynebacterium* sp. microorganism may be used as a seed culture medium, a main culture medium, or both. The details regarding the culture medium composition for a *Corynebacterium* sp. microorganism, seed culture medium, and main culture medium are as described above.

The culture containing a basic amino acid described above may have a sulfate concentration (g/L) in the medium reduced by 5% or more, for example, 5 to 60%, 5 to 55%, 5 to 50%, 5 to 55%, or 5 to 40%, at the end of the main culture, and/or have purity of the basic amino acid (for example, L-lysine, L-arginine, and/or L-histidine) in the medium increased by about 1% or more, about 1.5% or more, about 2% or more, about 3% or more, about 5% or more (the upper limit has no special restriction, and for example, it may be about 30% or less, about 20% or less, about 10% or less, about 8% or less, or about 7% less), at the end of the main culture, by using the seed culture medium described above and/or main culture medium described above , that is, by using the seed culture medium composition and/or main culture medium comprising the 'other ammonium-containing compound' described above, in the seed culture step and/or main culture step, compared to cases of using a culture medium that comprise none of ammonium-containing compound selected from the group consisting of ammonium succinate, ammonium phosphate, and ammonium acetate, more specifically, a culture medium comprising ammonium derived from the 'other ammonium-containing compound' in an amount of less than 0.05 mol, based on 1 mol of ammonium derived from ammonium sulfate, or a culture medium in which one or more of [Equation 1] to [Equation 4] described above is less than 2%, less than 3%, less than 4%, less than 5%, less than 6%, less than 6.5%, or less than 7% (including cases of comprising none of 'other ammonium-containing compound' described above) (See Example 2 and Table 2).

The culture containing a basic amino acid may have one or more characteristics selected from the following:

(a) a reduction in the sulfate concentration (g/L) in the medium at the end of seed culture by 10% or more, compared to a case in which a seed culture medium comprises ammonium derived from one or more selected from ammonium succinate, ammonium phosphate, and ammonium acetate in an amount of less than 0.05 mol, based on 1 mol of ammonium derived from ammonium sulfate;

(b) a reduction in the sulfate concentration (g/L) in the medium at the end of main culture by 5% or more, compared to a case in which a main culture medium comprises ammonium derived from one or more selected from ammonium succinate, ammonium phosphate, and ammonium acetate of less than 0.05 mol based on 1 mol of ammonium derived from ammonium sulfate; and

(c) an increase in purity of a basic amino acid in a medium at the end of main culture by 1% or more, compared to a case in which a main culture medium comprises ammonium derived from one or more selected from ammonium succinate, ammonium phosphate, and ammonium acetate of less than 0.05 mol based on 1 mol of ammonium derived from ammonium sulfate.

Method for preparing a culture medium composition for a *Corynebacterium* sp. microorganism

Other embodiment provides a method for preparing a culture medium composition for a *Corynebacterium* sp.

microorganism,
comprising adjusting contents of ammonium sulfate, and other ammonium-containing compounds (for example, one or more selected from the group consisting of ammonium succinate, ammonium phosphate, and ammonium acetate) in the culture medium composition for a *Corynebacterium* sp. microorganism so that the value of the following [Equation 1] is 2% or more: :

[(a sum of moles (mol) of ammonium derived from ammonium succinate, ammonium phosphate, and ammonium acetate)/(a sum of moles (mol) of ammonium derived from ammonium sulfate, ammonium succinate, ammonium phosphate, and ammonium acetate)]*100(%).    [Equation 1]

The medium prepared by the method is characterized in that the purity of the basic amino acid in the medium is increased by 1% or more at the end of culturing a microorganism having production ability of a basic amino acid, compared to a medium with the value of Equation 1 described above of less than 2%, or a medium having a value outside the range of 2% to 70%.

Accordingly, other embodiment provides a method for improving the purity of a basic amino acid of a culture medium for a *Corynebacterium* sp. microorganism comprising adjusting contents of ammonium sulfate, and other ammonium-containing compounds (for example, one or more selected from the group consisting of ammonium succinate, ammonium phosphate, and ammonium acetate) in the culture medium composition for a *Corynebacterium* sp. microorganism so that the value of the [Equation 1] described above is 2% or more.

The details regarding the *Corynebacterium* sp. microorganism, and the basic amino acid are as described above.

The culture medium composition for a *Corynebacterium* sp. microorganism may be used as a seed culture medium, a main culture medium, or both, and the details regarding the culture medium composition for a *Corynebacterium* sp. microorganism, seed culture medium, and main culture medium are as described above.

Use for producing a basic amino acid

Other embodiment provides uses of ammonium sulfate and one or more selected from the group consisting of ammonium succinate, ammonium phosphate, and ammonium acetate, for producing a basic amino acid, for increasing production (produced amount) of a basic amino acid, for increasing purity of a basic amino acid, for preparing a culture medium composition for a *Corynebacterium* sp. microorganism, and/or for preparing a medium composition for producing a basic amino acid.

The amount of ammonium sulfate and one or more selected from the group consisting of and ammonium succinate, ammonium phosphate, and ammonium acetate may be set such that the value of the following [Equation 1] of 2% or more:

[(a sum of moles (mol) of ammonium derived from ammonium succinate, ammonium phosphate, and ammonium acetate)/(a sum of moles (mol) of ammonium derived from ammonium sulfate, ammonium succinate, ammonium phosphate, and ammonium acetate)]*100(%).    [Equation 1]

Another embodiment provides uses of the composition comprising ammonium sulfate and one or more selected from the group consisting of ammonium succinate, ammonium phosphate, and ammonium acetate for producing a basic amino acid, increasing production (produced amount) of a basic amino acid, for increasing purity of a basic amino acid, for preparing a culture medium composition for a *Corynebacterium* sp. microorganism, and/or for preparing a medium composition for producing a basic amino acid.

The composition may have the value of the following [Equation 1] of 2% or more:

[(a sum of moles (mol) of ammonium derived from ammonium succinate, ammonium phosphate, and ammonium acetate)/(a sum of moles (mol) of ammonium derived from ammonium sulfate, ammonium succinate, ammonium phosphate, and ammonium acetate)]*100(%).    [Equation 1]

In the use of the present application, the details regarding ammonium sulfate, and ammonium succinate, ammonium phosphate, and ammonium acetate, the components of the seed culture medium and main culture medium, basic amino acid production, an increase in basic amino acid production (produced amount), an increase in the purity of basic amino acid, and the like are as described earlier in other embodiments.

Other embodiment provides a composition, method, product, process, or use characterized by one or more element disclosed in the present application.

[ADVANTAGEOUS EFFECTS]

In the present application, the purposes are to significantly increase the stability of the fermentation process and improve the purity of the fermented solution to enhance the product quality, compared to the conventional technology in which the entire amount in the medium is added as ammonium sulfate, by adding an ammonium substitute (ammonium-containing compound comprising an anion other than sulfate ion as an anionic counterion).

[MODE FOR INVENTION]

Hereinafter, the present application will be described by examples in more detail. However, the following examples are merely preferred embodiments to illustrate the present invention, and therefore, are not intended to limit the scope of the present application. Meanwhile, technical matters not described in the present description can be sufficiently understood and easily implemented by those skilled in the art of the present application or in similar technical fields.

**Reference Example: Obtaining the inoculum**

Inoculum preparation of coryneform microorganisms having lysine production ability was conducted by solid plate medium and flask culture, and a seed culture step was performed in a 5L fermenter, and a main culture step was performed in a 30L fermenter, respectively.

*Corynebacterium glutamicum* CJ3P (US 9556463 B2) stored in a deep-freezer of - 80°C in the form of Glycerol stock (GS) was taken in an amount of 50 mL and inoculated into a solid plate medium prepared based on the following medium composition by a streaking method, and cultured in a 30°C incubator for about 24 hours.

After that, in order to perform flask shaking culture, a liquid medium prepared based on the following flask shaking culture medium composition was prepared by 100 mL, and added to a 500 mL shaking flask containing a baffle to perform sterilization by using an autoclave at 121°C for 30 minutes. To perform shaking flask inoculation, the work was performed in a clean bench. After suitably taking colonies by using a disposable inoculating loop (100 mL loop) from the cultured solid plate, and then aseptically inoculated into the prepared shaking culture medium. The inoculated 500 mL shaking flask was cultured at 200 rpm, 30°C for about 10 hours.

For the seed culture step, the culture solution in the shaking flask in which inoculum preparation was conducted at 30°C as above, was inoculated into a 5 L fermenter containing the medium prepared based on the following seed culture medium composition at a ratio of 3.0%(v/v). The culture was carried out in a batch fermentation mode under conditions of pH 6.5-8.5, agitation speed of 800 rpm, and aeration rate of 1 vvm for approximately 20 hours.

1) Solid plate medium composition
Glucose 10.0 g/L, bacto tryptone 5.0 g/L, bacto yeast extract 5.0 g/L, NaCl 2.5 g/L, urea 2.0 g/L

2) Flask shaking culture medium composition
Glucose 10.0 g/L. bacto tryptone 5.0 g/L, bacto yeast extract 5.0 g/L, ammonium sulfate 10.0 g/L, urea 2.0 g/L, $KH_2PO_4$ 5.0 g/L, $K_2HPO_4$ 10.0 g/L, $MgSO_4$ $7H_2O$ 0.5 g/L

3) Fermenter (5 L) seed culture medium composition
Glucose 50.00g/L, antifoaming agent (fatty acid ester series) 1 mL/L, corn steep liquor 10.0 g/L, biotin 1.0 mg/L, thiamine 10.0 mg/L, pantothenic acid 10.0 mg/L, niacinamide 10.0 mg/L

In the examples below, the term "ammonium sulfate substitute" refers to an ammonium-containing compound comprising an anion other than a sulfate ion as an anionic counterion.

**Example 1: Addition of ammonium sulfate substitute to fermentation medium in seed culture step and fermentation performance according thereto**

**Example 1-1: Application of ammonium acetate in seed culture step**

Into a 5 L fermenter containing the "fermenter (5 L) seed culture medium composition" described in the Reference Example, to which 12.75 g/L of ammonium sulfate, 4.96 g/L of ammonium acetate, and 0.86 g/L of phosphoric acid were added, the inoculum culture obtained from the flask shaking culture of the Reference Example was inoculated at a ratio of 3.0% (v/v). In the present Example, a portion of ammonium sulfate added to the control medium of Comparative Example 1 was replaced with ammonium acetate. The replacement rate of ammonium sulfate was 25.0%, and the equation thereof is as follows (nitrogen weight per 1g of ammonium sulfate: 0.212g; nitrogen weight per 1g of ammonium acetate (ammonium substitute): 0.182g).

[(a sum of moles (mol) of ammonium derived from ammonium succinate, ammonium phosphate, and ammonium acetate)/(a sum of moles (mol) of ammonium derived from ammonium sulfate, ammonium     [Equation 1] succinate, ammonium phosphate, and ammonium acetate)]*100(%).

Culture was carried out in a batch fermentation mode under conditions of pH 6.5-8.5, agitation speed of 450 rpm, aeration rate of 1 vvm for 17.1 hours. When needed, pH was maintained in an appropriate range by addition of ammonia gas. Samples were obtained at intervals of approximately 2-3 hours, and at the time when residual sugar was depleted, the culture was terminated.

At the end of the culture, the cell density (OD) was 26.1, and the specific growth rate (/h) was 0.150. The residual sulfate concentration was reduced to 78.0% of the concentration of the control medium (Comparative Example 1), but the cell density was relatively low at the level of 58.4% of the control, indicating a decrease in cell activity (See Table 1).

**Example 1-2: Application of ammonium acetate and ammonium succinate in the seed culture step -1**

Into a 5 L fermenter containing the "fermenter (5 L) seed culture medium composition" described in Reference Example above, to which 12.75g/L of ammonium sulfate, 3.31g/L of ammonium acetate, 1.63g/L of ammonium succinate, 0.86g/L and phosphoric acid were added, the inoculum culture obtained from the flask shaking culture of Reference Example above was inoculated at a ratio of 3.0% (v/v). In the present Example, a portion of ammonium sulfate added to the control medium of Comparative Example 1 was replaced with ammonium acetate and ammonium succinate. The replacement rate of ammonium sulfate was 25.0% (See Example 1-1 for the equation of the replacement rate of ammonium sulfate; nitrogen weight per 1g of ammonium sulfate: 0.212g; nitrogen weight per 1g of ammonium acetate (ammonium substitute): 0.182g; nitrogen weight per 1g of ammonium succinate (ammonium substitute): 0.184g).

Culture was carried out in a batch fermentation mode under conditions of pH 6.5-8.5, agitation speed of 450 rpm, aeration rate of 1 vvm for about 16.0 hours. When needed, pH was maintained in an appropriate range by addition of ammonia gas. Samples were obtained at intervals of approximately 2-3 hours, and at the time when residual sugar was depleted, the culture was terminated.

At the end of the culture, the cell density (OD) was 40.8, and the specific growth rate (/h) was 0.170. The residual sulfate concentration was reduced to 77.6% of the concentration of the control medium (Comparative Example 1). While the cell density of Example 1-1 showed significantly reduced activity of 58.4% of that in Comparative Example 1, the cell density of Example 1-2 was 91.3% of that in Comparative Example 1, showing an improved result of 32.9%p compared to Example 1-1. In addition, the specific growth was improved by 12.9% compared to Example 1-1. These results suggest that reducing the amount of ammonium acetate, compared to the amount used in Example 1-1, may improve cell density and specific growth rate. It also indicates that even when ammonium sulfate is partially replaced with ammonium acetate and ammonium succinate, comparable or superior culture performance can be achieved. (See Table 1.)

**Example 1-3: Application of ammonium acetate and ammonium succinate in the seed culture step -2**

Into a 5 L fermenter containing the "fermenter (5 L) seed culture medium composition" described in Reference Example above, to which 12.75g/L of ammonium sulfate, 1.65g/L of ammonium acetate, 3.26g/L of ammonium succinate, and 0.86g/L of phosphoric acid were added, the inoculum culture obtained from the flask shaking culture of the Reference Example was inoculated at a ratio of 3.0% (v/v). In the present Example, a portion of ammonium sulfate added to the control medium of Comparative Example 1 was replaced with ammonium acetate and ammonium succinate, and the replacement rate of ammonium sulfate was 25.0% (See Example 1-1 for the equation of the replacement rate of ammonium sulfate; nitrogen weight per 1g of ammonium sulfate: 0.212g; nitrogen weight per 1g of ammonium acetate (ammonium substitute): 0.182g; nitrogen weight per 1g of ammonium succinate (ammonium substitute): 0.184g).

Culture was carried out in a batch fermentation mode under conditions of pH 6.5-8.5, agitation speed of 450 rpm, aeration rate of 1 vvm for 16.3 hours. When needed, pH was maintained in an appropriate range by addition of ammonia gas. Samples were obtained at intervals of approximately 2-3 hours, and at the time when residual sugar was depleted, the culture was terminated.

At the end of the culture, the cell density (OD) was 41.3, and the specific growth rate (/h) was 0.179. The residual sulfate was reduced to 78.1% of the concentration of the control medium (Comparative Example 1). While the cell density of Example 1-1 showed significantly reduced activity of 58.4% of that Comparative Example 1, the cell density of Example 1-3 was 92.4% of that Comparative Example 1, showing an improved result of 34.0%p compared to Example 1-1 and 1.1%p compared to Example 1-2. In addition, the specific growth rate was improved by 19.2% compared to Example 1-1 and by 5.6% compared to Example 1-2. These results suggest that a gradual reduction in the amount of ammonium acetate, compared to the level used in Example 1-1, leads to a stepwise improvement in both cell density and specific growth rate. This also demonstrates that even with partial replacement of ammonium sulfate by ammonium acetate and ammonium succinate, comparable or superior culture performance can be achieved. (See Table 1.)

**Example 1-4: Application of ammonium succinate in the seed culture step**

Into a 5 L fermenter containing the "fermenter (5 L) seed culture medium composition" described in Reference Example above, to which 12.80 g/L of ammonium sulfate, 4.89 g/L of ammonium succinate, and 0.86 g/L of phosphoric acid were added, the inoculum culture obtained from the flask shaking culture of the Reference Example was inoculated at a ratio of 3.0% (v/v). In the present Example, a portion of ammonium sulfate added to the control medium of Comparative Example 1 was replaced with ammonium succinate, and the replacement rate of ammonium sulfate was 24.9% (See Example 1-1 for the equation of the replacement rate of ammonium sulfate; nitrogen weight per 1g of ammonium sulfate: 0.212g; nitrogen weight per 1g of ammonium succinate (ammonium substitute): 0.184g).

Culture was carried out in a batch fermentation mode under conditions of pH 6.5-8.5, agitation speed of 450 rpm, aeration rate of 1 vvm for 17.0 hours. When needed, pH was maintained in an appropriate range by addition of ammonia gas. Samples were obtained at intervals of approximately 2-3 hours, and at the time when residual sugar was depleted, the culture was terminated.

At the end of the culture, the cell density (OD) was 44.8, and the specific growth rate (/h) was 0.183. The residual sulfate concentration was reduced to 76.7% compared to the concentration of the control medium (Comparative Example 1), Since both the cell density and the specific growth rate showed comparable or superior results, it indicates that partial replacement of ammonium sulfate with ammonium succinate can still achieve comparable or better culture performance. (See Table 1.)

**Example 1-5: Application of ammonium phosphate in the seed culture step**

Into a 5 L fermenter containing the "fermenter (5 L) seed culture medium composition" described in Reference Example above, to which 15.70g/L of ammonium sulfate, and 1.31g/L of ammonium phosphate were added, the inoculum culture obtained from the flask shaking culture of the Reference Example was inoculated at a ratio of 3.0% (v/v). In the present Example, a portion of ammonium sulfate added to the control medium of Comparative Example 1 was replaced with ammonium phosphate, and the replacement rate of ammonium sulfate was 7.7% (See Example 1-1 for the equation of the replacement rate of ammonium sulfate; nitrogen weight per 1g of ammonium sulfate: 0.212g; nitrogen weight per 1g of ammonium phosphate (ammonium substitute): 0.212g).

Culture was carried out in a batch fermentation mode under conditions of pH 6.5-8.5, agitation speed of 450 rpm, aeration rate of 1 vvm for 16.6 hours. When needed, pH was maintained in an appropriate range by addition of ammonia gas. Samples were obtained at intervals of approximately 2-3 hours, and at the time when residual sugar was depleted, the culture was terminated.

At the end of the culture, the cell density (OD) was 46.2, and the specific growth rate (/h) was 0.186. The residual sulfate concentration was reduced to 86.0% compared to the concentration of the control medium (Comparative Example 1). Since both the cell density and specific growth rate showed comparable or superior results, it indicates that partial replacement of ammonium sulfate with ammonium phosphate can achieve comparable or better culture performance. (See Table 1.)

**Comparative Example 1: Fermentation with ammonium sulfate added in the seed culture step (replacement rate of ammonium sulfate: 0%)**

Into a 5 L fermenter containing the "fermenter (5 L) seed culture medium composition" described in the Reference Example, to which 17.0 g/L of ammonium sulfate and 0.86 g/L of phosphoric acid were added, the inoculum culture obtained from the flask shaking culture of the Reference Example was inoculated at a ratio of 3.0% (v/v). Culture was carried out in a batch fermentation mode under conditions of pH 6.5-8.5, agitation speed of 450 rpm, aeration rate of 1 vvm for 17.1 hours. When needed, pH was maintained in an appropriate range by addition of ammonia gas. Samples were obtained at intervals of approximately 2-3 hours, and at the time when residual sugar was depleted, the culture was terminated.

At the end of the culture, the results that the cell density (OD) was 44.7, and the specific growth rate (/h) was 0.179. (See Table 1).

## Results

The results obtained from Examples 1-1 to 1-5 and Comparative Example 1 were described in Table 1 below:

[Table 1]

| Item | Unit | Example 1-1 | Example 1-2 | Example 1-3 | Example 1-4 | Example 1-5 | Comparative Example 1 |
|---|---|---|---|---|---|---|---|
| Fermentation time | hr | 17.1 | 16.0 | 16.3 | 17.0 | 16.6 | 17.1 |
| Cell density | OD | 26.1 | 40.8 | 41.3 | 44.8 | 46.2 | 44.7 |
| Specific growth rate | /h | 0.150 | 0.170 | 0.179 | 0.183 | 0.186 | 0.179 |
| Residual sugar | g/L | 22.8 | 0.0 | 0.0 | 0.0 | 0.0 | 0.0 |
| Sulfate* | % | 78.0 | 77.6 | 78.1 | 76.7 | 86.0 | 100.0 |
| (* Nomalization result values based on 100% of the results of Comparative Example 1; Cell density : measured as optical density (OD) at 562 nm using a spectrophotometer; Specific growth rate = (In(dry weight at time tf)-In(dry weight at time t0))/(tf-t0) (tf: final time; t0: start time); Dry weight : calculated by cell density (OD*0.25=g/L)) | | | | | | | |

In summary of the experimental results of Table 1,

in order to improve the purity of the final product, it is important to reduce by-products in the fermented solution obtained in the main culture step, but it is essential to reduce residual by-products in the fermented solution in the seed culture step required for main culture inoculation.

In the results of Example 1-1 where ammonium acetate was used, a 16.0% decrease in specific growth rate and a 41.6% decrease in cell density were observed compared to Comparative Example 1. Since the ultimate purpose of the seed culture step is to activate seed and increase the cell number in a short period of time, it was confirmed that using a high concentration of ammonium acetate with a replacement rate of ammonium sulfate of 25.0% is not suitable for application in the seed culture, despite achieving a 22.0% reduction in residual sulfate.In the results of Example 1-2 where ammonium acetate and ammonium succinate were used, the amount of ammonium acetate added was reduced, and the amount of ammonium succinate was increased, compared to Example 1-1, while maintaining the same replacement rate of ammonium sulfate of 25.0%. Compared to Comparative Example 1, a 5.2% decrease in specific growth rate and an 8.7% decrease in cell density were observed, suggesting potential for improvement in both parameters. Accordingly, in the culture process using ammonium acetate (66.6% of Example 1-1 added) and ammonium succinate, the residual sulfate was reduced by 22.4%, and comparable culture indexes were achieved, suggesting the applicability in the seed culture step.

In the results of Example 1-3 where ammonium acetate and ammonium succinate were used, the amount of ammonium acetate added was reduced, and the amount of ammonium succinate was increased compared to Example 1-2, while maintaining the same replacement rate of ammonium sulfate of 25.0%. Compared to Comparative Example 1, a comparable specific growth rate and a 7.6% decrease in cell density were observed, suggesting the possibility of improvement of the cell density and specific growth rate. Accordingly, in the culture process using ammonium succinate (33.3% of Example 1-1 added) and ammonium succinate, the residual sulfate was reduced by 21.9%, and comparable culture indexes were confirmed, suggesting the applicability in the seed culture step.

In the results of Example 1-4 where ammonium succinate was used, comparable or superior outcomes were observed in terms of fermentation time, cell density, and specific growth rate compared to Comparative Example 1. In addition, the residual sulfate concentration was reduced by 23.3%, confirming that ammonium succinate can be applied as a substitute for ammonium sulfate at a replacement rate of 24.9% in the seed culture step.

In the results Example 1-5 wherein ammonium phosphate was used, comparable or superior outcomes were observed in terms of fermentation time, cell density, and specific growth rate compared to Comparative Example 1. In addition, the residual sulfate concentration was reduced by 14.0%, confirming that ammonium phosphate can be applied as a substitute for ammonium sulfate at a replacement rate of 7.7% in the seed culture step.

**Example 2: Addition of ammonium sulfate substitute to fermentation medium in seed culture or main culture step and fermentation performance according thereto**

**Example 2-1: Application of ammonium sulfate substitute combination to the fermentation medium in seed culture step -1**

Into a 5 L fermenter containing the "fermenter (5 L) seed culture medium composition" described in the Reference Example, to which 11.50 g/L of ammonium sulfate, 4.89 g/L of ammonium succinate, and 1.31 g/L of ammonium phosphate were added, the inoculum culture obtained from the flask shaking culture of the Reference Example was inoculated at a ratio of 3.0% (v/v). (replacement rate of ammonium sulfate 32.6%; See Example 1-1 for the equation for the replacement rate of ammonium sulfate; nitrogen weight per 1g of ammonium sulfate : 0.212g, nitrogen weight per 1g of ammonium succinate (ammonium substitute) : 0.184g, nitrogen weight per 1g of ammonium phosphate (ammonium substitute) : 0.212g).

Culture was carried out in a batch fermentation mode under conditions of pH 6.5-8.5, agitation speed of 450 rpm, aeration rate of 1 vvm for about 16 hours. When needed, pH was maintained in an appropriate range by addition of ammonia gas. Samples were obtained at intervals of approximately 2-3 hours, and at the time when residual sugar was depleted, the culture was terminated.

A 30 L fermenter containing the main culture medium composition described below, to which 13.00g/L of ammonium sulfate, and 0.86g/L of phosphoric acid were added, was inoculated with the seed culture solution obtained in the seed culture step at a ratio of 20% (v/v).

Fermenter (30 L) main culture medium composition

Glucose 25.00g/L, antifoaming agent 1 mL/L, corn steep liquor 10.0 g/L, biotin 1.0 mg/L, thiamine 10.0 mg/L, pantothenic acid 10.0 mg/L, niacinamide 10.0 mg/L

In the present Example, a portion of ammonium sulfate added to the seed culture medium of Comparative Example 2 below was replaced with ammonium succinate and ammonium phosphate, and the replacement rate of ammonium sulfate of the main culture medium was 0.0%. The total replacement ratio of the seed culture medium and main culture medium is 6.8%.

In the main culture step of the present Example, in a fed-batch fermentation mode, culture was conducted under conditions of pH 6.5-8.5, agitation speed of 450 rpm, aeration rate of 1 vvm. When needed, pH was maintained in an appropriate range by addition of ammonia gas.

When initial carbon sources of the medium were depleted, a feeding medium comprising 40.0%wt glucose was introduced to maintain residual sugar at 0.5-1.0wt%. Samples were obtained at intervals of approximately 2-3 hours, and the nitrogen content (AN: ammonium nitrogen, g/kg) was measured using a Kjeldahl analyzer (Foss Kjeltec 8400). When the AN value was less than 1.0 g/kg, nitrogen sources were further supplied by addition of ammonia gas.

At the end of the culture (i.e., when the sugar was depleted), the residual sugar was not present, and the fermentation time was 29.5 hours. At the end of the culture, the residual sulfate concentration in the medium, derived from ammonium sulfate-normalized based on the result of Comparative Example 2 (set as 100%)-was 92.0%, and the L-lysine purity obtained from the main culture-also normalized-was 102.5%. Compared to the result of Comparative Example 2, this corresponds to an 8.0% reduction in residual sulfate and a 2.5% improvement in L-lysine purity. (See Table 2.)

**Example 2-2: Application of ammonium sulfate substitute combination to the fermentation medium in seed culture step -2**

Into a 5 L fermenter containing the "fermenter (5 L) seed culture medium composition" described in the Reference Example, to which 14.17 g/L of ammonium sulfate, 2.0 g/L of ammonium succinate, and 1.6 g/L of ammonium phosphate (replacement rate of ammonium sulfate: 19.1%) were added, the inoculum culture obtained from the flask shaking culture of the Reference Example was inoculated at a ratio of 3.0% (v/v).Culture was carried out in a batch fermentation mode under conditions of pH 6.5-8.5, agitation speed of 450 rpm, aeration rate of 1 vvm for about 17 hours. When needed, pH was maintained in an appropriate range by addition of ammonia gas. Samples were obtained at intervals of approximately 2-3 hours, and at the time when residual sugar was depleted, the culture was terminated.

A 30 L fermenter containing the main culture medium composition of Example 2-1, to which 13.0 g/L of ammonium sulfate and 0.86 g/L of phosphoric acid were added, was inoculated with the seed culture solution obtained in the seed culture step at a ratio of 20% (v/v). The replacement rate of ammonium sulfate of the main culture medium was 0.0%, and the total replacement rate of ammonium sulfate of the seed culture medium and main culture medium was 4.0%.

In the main culture step of the present Example, in a fed-batch fermentation mode, culture was conducted under conditions of pH 6.5-8.5, agitation speed of 450 rpm, aeration rate of 1 vvm. When needed, pH was maintained in an appropriate range by addition of ammonia gas.

When initial carbon sources of the medium were depleted, a feeding medium comprising 40.0%wt glucose was introduced to maintain residual sugar at 0.5-1.0wt%. Samples were obtained at intervals of approximately 2-3 hours, and

EP 4 763 974 A1

the nitrogen content (AN: ammonium nitrogen, g/kg) was measured using a Kjeldahl analyzer (Foss Kjeltec 8400). When the AN value was less than 1.0 g/kg, nitrogen sources were further supplied by addition of ammonia gas.

At the end of the culture, the residual sugar was not present, and the fermentation time was 29.0 hours. The normalized residual sulfate concentration derived from ammonium sulfate and the normalized purity obtained in the main culture step were 94.0%, 102.1%, respectively, indicating a 6.0% reduction in residual sulfate and a 2.1% improvement in purity compared to the result of Comparative Example 2 (see Table 2).

**Example 2-3: Application of ammonium sulfate substitute combination to the fermentation medium in main culture step**

Into a 5 L fermenter containing the "fermenter (5 L) seed culture medium composition" described in the Reference Example, to which 17 g/L of ammonium sulfate and 0.86 g/L of phosphoric acid (replacement rate of ammonium sulfate: 0%) were added, the inoculum culture obtained from the flask shaking culture of the Reference Example was inoculated at a ratio of 3.0% (v/v).Culture was carried out in a batch fermentation mode under conditions of pH 6.5-8.5, agitation speed of 450 rpm, aeration rate of 1 vvm for about 17 hours. When needed, pH was maintained in an appropriate range by addition of ammonia gas. Samples were obtained at intervals of approximately 2-3 hours, and at the time when residual sugar was depleted, the culture was terminated.

A 30 L fermenter containing the main culture medium composition of Example 2-1, to which 6.10 g/L of ammonium sulfate, 3.28 g/L of ammonium acetate, 3.24 g/L of ammonium succinate, and 1.31 g/L of ammonium phosphate were added, was inoculated with the seed culture solution obtained in the seed culture step at a ratio of 20% (v/v). In the present Example, a portion of ammonium sulfate added to the main culture medium of Comparative Example 2 was replaced with ammonium acetate, ammonium succinate, and ammonium phosphate. The replacement rate of ammonium sulfate of the main culture medium was 53.2%, and the total replacement rate of ammonium sulfate of the seed culture medium and main culture medium was 42.2%.

In the main culture step of the present Example, in a fed-batch fermentation mode, culture was conducted under conditions of pH 6.5-8.5, agitation speed of 450 rpm, aeration rate of 1 vvm. When needed, pH was maintained in an appropriate range by addition of ammonia gas.

When initial carbon sources of the medium were depleted, a feeding medium comprising 40.0%wt glucose was introduced to maintain residual sugar at 0.5-1.0wt%. Samples were obtained at intervals of approximately 2-3 hours, and the nitrogen content (AN: ammonium nitrogen, g/kg) was measured using a Kjeldahl analyzer (Foss Kjeltec 8400). When the AN value was less than 1.0 g/kg, nitrogen sources were further supplied by addition of ammonia gas.

At the end of the culture, the residual sugar was not present, and the fermentation time was 30.1 hours. The normalized residual sulfate concentration derived from ammonium sulfate and the normalized purity obtained in the main culture step were 72.0%, 102.9%, respectively, indicating a 28.0% reduction in residual sulfate and a 2.9% improvement in purity compared to the result of Comparative Example 2 (see Table 2).

**Example 2-4: Application of ammonium sulfate substitute combination to the fermentation medium in seed and main culture step -1**

Into a 5 L fermenter containing the "fermenter (5 L) seed culture medium composition" described in the Reference Example, to which 11.50 g/L of ammonium sulfate, 4.89 g/L of ammonium succinate, and 1.31 g/L of ammonium phosphate (replacement rate of ammonium sulfate: 32.6%) were added, the inoculum culture obtained from the flask shaking culture of the Reference Example was inoculated at a ratio of 3.0% (v/v).

Culture was carried out in a batch fermentation mode under conditions of pH 6.5-8.5, agitation speed of 450 rpm, aeration rate of 1 vvm for about 17 hours. When needed, pH was maintained in an appropriate range by addition of ammonia gas. Samples were obtained at intervals of approximately 2-3 hours, and at the time when residual sugar was depleted, the culture was terminated.

A 30 L fermenter containing the main culture medium composition of Example 2-1, to which 6.10 g/L of ammonium sulfate, 3.28 g/L of ammonium acetate, 3.24 g/L of ammonium succinate, and 1.31 g/L of ammonium phosphate (replacement rate of ammonium sulfate: 53.2%) were added, was inoculated with the seed culture solution obtained in the seed culture step at a ratio of 20% (v/v).

In the present Example, a portion of ammonium sulfate added to the seed culture medium of Comparative Example 2 was replaced with ammonium succinate and ammonium phosphate, and a portion of ammonium sulfate added to the main culture medium of Comparative Example 2 was replaced with ammonium acetate, ammonium succinate, and ammonium phosphate. The total replacement rate of ammonium sulfate of the seed culture medium and main culture medium was 48.9%.

In the main culture step of the present Example, in a fed-batch fermentation mode, culture was conducted under conditions of pH 6.5-8.5, agitation speed of 450 rpm, aeration rate of 1 vvm for about 30 hours. When needed, pH was maintained in an appropriate range by addition of ammonia gas.

When initial carbon sources of the medium were depleted, a feeding medium comprising 40.0%wt glucose was introduced to maintain residual sugar at 0.5-1.0wt%. Samples were obtained at intervals of approximately 2-3 hours, and the nitrogen content (AN: ammonium nitrogen, g/kg) was measured using a Kjeldahl analyzer (Foss Kjeltec 8400). When the AN value was less than 1.0 g/kg, nitrogen sources were further supplied by addition of ammonia gas.

At the end of the culture, the residual sugar was not present, and the fermentation time was 29.9 hours. The normalized residual sulfate concentration derived from ammonium sulfate and the normalized purity obtained in the main culture step were 60.0%, 102.9%, respectively, and compared to the results of Comparative Example 2, indicating a 40.0% reduction in residual sulfate and a 2.9% improvement in purity compared to the result of Comparative Example 2 (see Table 2).

**Example 2-5: Application of ammonium sulfate substitute combination to the fermentation medium in seed and main culture step -2**

Into a 5 L fermenter containing the "fermenter (5 L) seed culture medium composition" described in the Reference Example, to which 11.50 g/L of ammonium sulfate, 4.89 g/L of ammonium succinate, and 1.31 g/L of ammonium phosphate (replacement rate of ammonium sulfate: 32.6%) were added, the inoculum culture obtained from the flask shaking culture of the Reference Example was inoculated at a ratio of 3.0% (v/v).

Culture was carried out in a batch fermentation mode under conditions of pH 6.5-8.5, agitation speed of 450 rpm, aeration rate of 1 vvm for about 17 hours. When needed, pH was maintained in an appropriate range by addition of ammonia gas. Samples were obtained at intervals of approximately 2-3 hours, and at the time when residual sugar was depleted, the culture was terminated.

A 30 L fermenter containing the main culture medium composition of Example 2-1, to which 3.80 g/L of ammonium sulfate, 3.28 g/L of ammonium acetate, 5.15 g/L of ammonium succinate, and 1.31 g/L of ammonium phosphate (replacement rate of ammonium sulfate: 69.3%) were added, was inoculated with the seed culture solution obtained in the seed culture step at a ratio of 20% (v/v).

In the present Example, a portion of ammonium sulfate added to the seed culture medium of Comparative Example 2 was replaced with ammonium succinate, and ammonium phosphate, and a portion of ammonium sulfate added to the main culture medium of Comparative Example 2 was replaced with ammonium acetate, ammonium succinate, and ammonium phosphate. The total replacement rate of ammonium sulfate of the seed culture medium and main culture medium was 61.4%.

In the main culture step of the present Example, in a fed-batch fermentation mode, culture was conducted under conditions of pH 6.5-8.5, agitation speed of 450 rpm, aeration rate of 1 vvm for about 30 hours. When needed, pH was maintained in an appropriate range by addition of ammonia gas.

When initial carbon sources of the medium were depleted, a feeding medium comprising 40.0%wt glucose was introduced to maintain residual sugar at 0.5-1.0wt%. Samples were obtained at intervals of approximately 2-3 hours, and the nitrogen content (AN: ammonium nitrogen, g/kg) was measured using a Kjeldahl analyzer (Foss Kjeltec 8400). When the AN value was less than 1.0 g/kg, nitrogen sources were further supplied by addition of ammonia gas.

At the end of the culture, the residual sugar was not present, and the fermentation time was 29.1 hours. The normalized residual sulfate concentration derived from ammonium sulfate and the normalized purity obtained in the main culture step were 48.0%, 105.4%, respectively, and compared to the results of Comparative Example 2, indicating a 52.0% reduction in residual sulfate and a 5.4% improvement in purity compared to the result of Comparative Example 2 (see Table 2).

**Example 2-6: Application of ammonium sulfate substitute combination to the fermentation medium in seed and main culture step -3**

Into a 5 L fermenter containing the "fermenter (5 L) seed culture medium composition" described in the Reference Example, to which 11.50 g/L of ammonium sulfate, 4.89 g/L of ammonium succinate, and 1.31 g/L of ammonium phosphate (replacement rate of ammonium sulfate: 32.6%) were added, an inoculum culture obtained by the method described below was inoculated at a ratio of 3.0% (v/v). In the present Example 2-6, the same flask shaking culture medium used in the Reference Example was employed, but a different Coryneform microorganism capable of producing lysine, identified by the deposit number KCCM11016P (Korean Patent Nos. 10-0159812 and 10-0397322), was used for the shaking culture. The inoculum culture was obtained by this method. The microorganism having the deposit number KCCM11016P was initially disclosed as KFCC10881 and was subsequently re-deposited with an international depository authority under the Budapest Treaty, where it was assigned the deposit number KCCM11016P.

Culture was carried out in a batch fermentation mode under conditions of pH 6.5-8.5, agitation speed of 450 rpm, aeration rate of 1 vvm for about 17 hours. When needed, pH was maintained in an appropriate range by addition of ammonia gas. Samples were obtained at intervals of approximately 2-3 hours, and at the time when residual sugar was depleted, the culture was terminated.

A 30 L fermenter containing the main culture medium composition of Example 2-1, to which 6.10 g/L of ammonium sulfate, 3.28 g/L of ammonium acetate, 3.24 g/L of ammonium succinate, and 1.31 g/L of ammonium phosphate (replacement rate of ammonium sulfate: 53.2%) were added, was inoculated with the seed culture solution obtained in the seed culture step at a ratio of 20% (v/v).

In the present Example, a portion of ammonium sulfate added to the seed culture medium of Comparative Example 2 was replaced with ammonium succinate, and ammonium phosphate, and a portion of ammonium sulfate added to the main culture medium of Comparative Example 2 was replaced with ammonium acetate, ammonium succinate, and ammonium phosphate. The total replacement rate of ammonium sulfate of the seed culture medium and main culture medium was 48.9%.

In the main culture step of the present Example, in a fed-batch fermentation mode, culture was conducted under conditions of pH 6.5-8.5, agitation speed of 450 rpm, aeration rate of 1 vvm for about 30 hours. When needed, pH was maintained in an appropriate range by addition of ammonia gas.

When initial carbon sources of the medium were depleted, a feeding medium comprising 40.0%wt glucose was introduced to maintain residual sugar at 0.5-1.0wt%. Samples were obtained at intervals of approximately 2-3 hours, and the nitrogen content (AN: ammonium nitrogen, g/kg) was measured using a Kjeldahl analyzer (Foss Kjeltec 8400). When the AN value was less than 1.0 g/kg, nitrogen sources were further supplied by addition of ammonia gas.

At the end of the culture, the residual sugar was not present, and the fermentation time was 28.6 hours. The normalized residual sulfate concentration derived from ammonium sulfate and the normalized purity obtained in the main culture step were 59.4%, 103.2%, respectively, and compared to the results of Comparative Example 2, indicating a 40.6% reduction in residual sulfate and a 3.2% improvement in purity compared to the result of Comparative Example 2 (see Table 2).

**Comparative Example 2: Addition of ammonium sulfate to media of seed and main culture step**

Into a 5 L fermenter containing the "fermenter (5 L) seed culture medium composition" described in the Reference Example, to which 17.00g/L of ammonium sulfate, and 0.86g/L of phosphoric acid were added, the inoculum culture obtained from the flask shaking culture of the Reference Example was inoculated at a ratio of 3.0% (v/v).

Culture was carried out in a batch fermentation mode under conditions of pH 6.5-8.5, agitation speed of 450 rpm, aeration rate of 1 vvm for about 17.0 hours. When needed, pH was maintained in an appropriate range by addition of ammonia gas. Samples were obtained at intervals of approximately 2-3 hours, and at the time when residual sugar was depleted, the culture was terminated.

A 30 L fermenter containing the main culture medium composition of Example 2-1, to which 13.00 g/L of ammonium sulfate and 0.86 g/L of phosphoric acid (replacement rate of ammonium sulfate: 69.3%) were added, was inoculated with the seed culture solution obtained in the seed culture step at a ratio of 20% (v/v).In the main culture step of the present Comparative Example 2, in a fed-batch fermentation mode, culture was conducted under conditions of pH 6.5-8.5, agitation speed of 450 rpm, aeration rate of 1 vvm for 30.6 hours. When needed, pH was maintained in an appropriate range by addition of ammonia gas.

When initial carbon sources of the medium were depleted, a feeding medium comprising 40.0%wt glucose was introduced to maintain residual sugar at 0.5-1.0wt%. Samples were obtained at intervals of approximately 2-3 hours, and the nitrogen content (AN: ammonium nitrogen, g/kg) was measured using a Kjeldahl analyzer (Foss Kjeltec 8400). When the AN value was less than 1.0 g/kg, nitrogen sources were further supplied by addition of ammonia gas.

**Results**

The results obtained from Examples 2-1 to 2-6 and Comparative Example 2 were described in Table 2 below:

[Table 2]

| Item | Unit | Example 2-1 | Example 2-2 | Example 2-3 | Example 2-4 | Example 2-5 | Example 2-6 | Comparative Example 2 |
|---|---|---|---|---|---|---|---|---|
| Fermentation time | hr | 29.5 | 29.0 | 30.1 | 29.9 | 29.1 | 28.6 | 30.6 |
| Residual sugar | g/L | 0.0 | 0.0 | 0.0 | 0.0 | 0.0 | 0.0 | 0.0 |

(continued)

| Item | Unit | Example 2-1 | Example 2-2 | Example 2-3 | Example 2-4 | Example 2-5 | Example 2-6 | Comparative Example 2 |
|---|---|---|---|---|---|---|---|---|
| Sulfate* | % | 92.0 | 94.0 | 72.0 | 60.0 | 48.0 | 59.4 | 100.0 |
| Lysine (Lys) | g/L | 218.9 | 216.9 | 214.2 | 217.0 | 221.5 | 217.3 | 219.4 |
| Purity* | % | 102.5 | 102.1 | 102.9 | 102.9 | 105.4 | 103.2 | 100.0 |
| (* Normalization result values based on 100% of the results of Comparative Example 2; | | | | | | | | |

$$Purity(\%) = [Lys\ content\ (g/kg) / solid\ content\ (g/kg)] * 100$$

The experiment results of Table 2 are summarized as follows:
In Example 2-1, culture was carried out by replacing ammonium sulfate added in the seed culture step with ammonium succinate and ammonium phosphate under the condition of the replacement rate of ammonium sulfate of 6.8%. While the fermentation time and lysine concentration were at comparable levels to the control, the normalized residual sulfate in the fermentation solution was reduced by 8.0%, leading to a 2.5% improvement in the normalized lysine purity.

In Example 2-2, culture was carried out by replacing ammonium sulfate added in the seed culture step with ammonium succinate and ammonium phosphate under the condition of the replacement rate of ammonium sulfate of 4.0%. While the fermentation time and lysine concentration were at comparable levels to the control, the normalized residual sulfate in the fermentation solution was reduced by 6.0%, leading to a 2.1% improvement in the normalized lysine purity.

In Example 2-3, culture was carried out by replacing ammonium sulfate added in the seed culture step with ammonium succinate and ammonium phosphate under the condition of the replacement rate of ammonium sulfate of 42.2%. While the fermentation time and lysine concentration were at comparable levels to the control, the normalized residual sulfate in the fermentation solution was reduced by 28.0%, leading to a 2.9% improvement in the normalized lysine purity.

In Example 2-4, culture was carried out by replacing ammonium sulfate added in the seed culture with ammonium succinate and ammonium phosphate, and replacing ammonium sulfate added in the main culture step with ammonium acetate, ammonium succinate, and ammonium phosphate, under the condition of the replacement rate of ammonium sulfate of 48.9%. While the fermentation time and lysine concentration were at comparable levels to the control, the normalized residual sulfate in the fermentation solution was reduced by 40.0%, leading to a 2.9% improvement in the normalized lysine purity.

In Example 2-5, culture was carried out by replacing ammonium sulfate added in the seed culture with ammonium succinate and ammonium phosphate, and replacing ammonium sulfate added in the main culture step with ammonium acetate, ammonium succinate, and ammonium phosphate, under the condition of the replacement rate of ammonium sulfate of 61.4%. While the fermentation time and lysine concentration were at comparable levels to the control, the normalized residual sulfate in the fermentation solution was reduced by 52.0%, leading to a 5.4% improvement in the normalized lysine purity.

In Example 2-6, KCCM11016P, which is a coryneform microorganism having the lysine production ability under the conditions of Reference Example and Example 2-4, was used. Culture was carried out by replacing ammonium sulfate added in the seed culture with ammonium succinate and ammonium phosphate, and replacing ammonium sulfate added in the main culture step with ammonium acetate, ammonium succinate, and ammonium phosphate, under the condition of the replacement rate of ammonium sulfate of 48.9%. As a result, while the fermentation time and lysine concentration were at comparable levels to the control, the normalized residual sulfate in the fermentation solution was reduced by 40.6%, leading to a 3.2% improvement in the normalized lysine purity. In addition, compared to the results of Example 2-4, the residual sulfate and normalized purity showed similar values to each other, confirming that even when the strain was changed, similar fermentation indicators and product quality were achieved under the same ammonium sulfate replacement rate.

In order to improve the purity of the final product, it is essential to improve the purity of the fermented solution acquired by minimizing ammonium sulfate-derived residual sulfate added to the medium. In the lysine granulation process without a separate ion exchange resin process, the problem of product purity decline due to fermented solution impurities occurs, but when a portion or total of ammonium sulfate of the seed culture medium and main culture step medium was replaced with ammonium acetate, ammonium succinate and ammonium phosphate by introducing the present technology, the anionic counterion was consumed during the culture, and the concentration of sulfate was reduced, and thus, the effect of improving the quality of the granule product was confirmed.

From the above description, those skilled in the art to which the present invention pertains will understand that the present invention can be implemented in other specific forms without changing the technical spirit or essential features thereof. In this regard, it should be understood that the examples described above are exemplary and not restrictive in all aspects. The scope of the present invention should be interpreted as including all changed or modified forms derived from the meaning and scope of the claims described below and equivalent concepts thereof rather than the detailed description above.

**Claims**

1.  A culture medium composition for a *Corynebacterium* sp. microorganism, comprising ammonium sulfate; and one or more ammonium-containing compounds selected from the group consisting of ammonium succinate, ammonium phosphate, and ammonium acetate,
    wherein the value of the following [Equation 1] is 2% or more:

    [(a sum of moles (mol) of ammonium derived from ammonium succinate, ammonium phosphate, and ammonium acetate)/(a sum of moles (mol) of ammonium derived from ammonium sulfate,    [Equation 1] ammonium succinate, ammonium phosphate, and ammonium acetate)]*100(%).

2.  The culture medium composition according to claim 1,
    wherein the value of the following [Equation 5] is 90% or less:

    [(moles (mol) of ammonium derived from ammonium acetate)/(a sum of moles (mol) of ammonium derived from ammonium succinate, ammonium phosphate, and ammonium acetate)]*100    [Equation 5] (%).

3.  The culture medium composition according to claim 1,
    wherein the sum of moles (mol) of ammonium derived from ammonium sulfate, ammonium succinate, ammonium phosphate, and ammonium acetate is 0.1 mol to 0.5 mol per 1L of the culture medium composition.

4.  The culture medium composition according to claim 1,

    wherein the culture medium composition, comprises one or more selected from the group consisting of
    0.05 mol to 1.2 mol of ammonium derived from ammonium succinate,
    0.01 mol to 0.5 mol of ammonium derived from ammonium phosphate, and
    0.05 mol to 1 mol of ammonium derived from ammonium acetate,
    based on 1 mol of ammonium derived from ammonium sulfate.

5.  The culture medium composition according to claim 1,
    wherein the culture medium composition has a pH of 6 to 9.

6.  The culture medium composition according to claim 1,
    wherein the *Corynebacterium* sp. microorganism is *Corynebacterium glutamicum* or *Corynebacterium stationis*.

7.  The culture medium composition according to claim 1,
    wherein the culture medium composition is a seed culture medium composition or a main culture medium composition.

8.  A method for producing a basic amino acid, comprising culturing a *Corynebacterium* sp. microorganism in the culture medium composition according to any one claim of claim 1 to claim 7.

9.  The method for producing a basic amino acid according to claim 8,
    wherein the culture medium composition is a seed culture medium composition.

10. The method for producing a basic amino acid according to claim 9,

further comprising culturing the *Corynebacterium* sp. microorganism by introducing the seed culture medium composition comprising the *Corynebacterium* sp. microorganism into a main culture medium.

11. The method for producing a basic amino acid according to claim 10, wherein the seed culture medium composition comprising the *Corynebacterium* sp. microorganism is introduced in an amount of 0.1L to 0.4L per 1L of the main culture medium.

12. The method for producing a basic amino acid according to claim 10, wherein a feeding medium is further introduced during culturing the *Corynebacterium* sp. microorganism by introducing the seed culture medium composition comprising the *Corynebacterium* sp. microorganism into a main culture medium.

13. The method for producing a basic amino acid according to claim 8, wherein the pH of the culture medium is maintained at 6 to 9 during the culturing.

14. The method for producing a basic amino acid according to claim 8, wherein the sulfate content at the end of the culturing is reduced, compared to a case of using a culture medium that comprises none of ammonium-containing compound selected from the group consisting of ammonium succinate, ammonium phosphate, and ammonium acetate.

15. The method for producing a basic amino acid according to claim 8, wherein the purity of the basic amino acid at the end of the culturing is increased, compared to a case of using a culture medium that comprises none of ammonium-containing compound selected from the group consisting of ammonium succinate, ammonium phosphate, and ammonium acetate.

**EP 4 763 974 A1**

## INTERNATIONAL SEARCH REPORT

| International application No. |
|---|
| **PCT/KR2025/013040** |

| A. | CLASSIFICATION OF SUBJECT MATTER |
|---|---|

**C12N 1/38**(2006.01)i; **C12N 1/20**(2006.01)i; **C12P 13/08**(2006.01)i; C12R 1/15(2006.01)n; C12R 1/185(2006.01)n

According to International Patent Classification (IPC) or to both national classification and IPC

| B. | FIELDS SEARCHED |
|---|---|

Minimum documentation searched (classification system followed by classification symbols)

C12N 1/38(2006.01); C12N 1/00(2006.01); C12N 1/20(2006.01); C12N 1/21(2006.01); C12N 15/77(2006.01); C12P 1/00(2006.01); C12P 13/08(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (MOIP internal) & keywords: 황산 암모늄(ammonium sulfate), 숙신산 암모늄(ammonium succinate), 인산 암모늄(ammonium phosphate), 초산 암모늄(ammonium acetate), 코리네박테리움속(Corynebacterium sp.), 배양 배지(culture medium), 염기성 아미노산(basic amino acid)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 112646844 A (TIANJIN UNIVERSITY OF SCIENCE & TECHNOLOGY et al.) 13 April 2021 (2021-04-13)<br>see paragraphs [0020], [0030]; example 1; and claims 1, 3. | 1,3-15 |
| Y | | 2 |
| Y | MOOSAVI-NASAB, M. et al. Fermentative Production of Lysine by Corynebacterium glutamicum from Different Carbon Sources. Iran Agricultural Research. 2007, vol. 25, no. 2 and vol. 26, nos. 1-2, pp. 99-106.<br>see abstract ; pages 101-102; and figure 1. | 2 |
| X | CN 111197021 A (JIANGNAN UNIVERSITY et al.) 26 May 2020 (2020-05-26)<br>see paragraphs [0026]-[0030]; and claims 9-10. | 1,6-8 |
| A | KR 10-2009-0023513 A (AJINOMOTO CO., INC.) 04 March 2009 (2009-03-04)<br>see entire document. | 1-15 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **21 November 2025** | **22 November 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

25

**INTERNATIONAL SEARCH REPORT**

International application No.

**PCT/KR2025/013040**

**C.    DOCUMENTS CONSIDERED TO BE RELEVANT**

| Category* | Citation of document, with indication, where appropriate, of the relevant passages | Relevant to claim No. |
|---|---|---|
| A | KR 10-2023-0029769 A (LONZA LTD.) 03 March 2023 (2023-03-03)<br>see entire document. | 1-15 |

### INTERNATIONAL SEARCH REPORT
**Information on patent family members**

International application No.

**PCT/KR2025/013040**

| Patent document cited in search report | | | Publication date (day/month/year) | Patent family member(s) | | | Publication date (day/month/year) |
|---|---|---|---|---|---|---|---|
| CN | 112646844 | A | 13 April 2021 | None | | | |
| CN | 111197021 | A | 26 May 2020 | CN | 111197021 | B | 21 September 2021 |
| KR | 10-2009-0023513 | A | 04 March 2009 | CN | 101111602 | A | 23 January 2008 |
| | | | | CN | 101111602 | B | 04 July 2012 |
| | | | | CN | 103088080 | A | 08 May 2013 |
| | | | | CN | 103088080 | B | 17 February 2016 |
| | | | | EP | 1813677 | A1 | 01 August 2007 |
| | | | | EP | 1813677 | A4 | 19 October 2011 |
| | | | | EP | 1813677 | B1 | 06 February 2019 |
| | | | | EP | 2818554 | A2 | 31 December 2014 |
| | | | | EP | 2818554 | A3 | 11 February 2015 |
| | | | | EP | 2818555 | A2 | 31 December 2014 |
| | | | | EP | 2818555 | A3 | 18 February 2015 |
| | | | | EP | 2818555 | B1 | 28 December 2022 |
| | | | | EP | 2818556 | A2 | 31 December 2014 |
| | | | | EP | 2818556 | A3 | 18 February 2015 |
| | | | | EP | 2818556 | B1 | 10 May 2023 |
| | | | | JP | 4844396 | B2 | 28 December 2011 |
| | | | | KR | 10-0921644 | B1 | 14 October 2009 |
| | | | | KR | 10-2007-0061587 | A | 13 June 2007 |
| | | | | US | 2007-0243590 | A1 | 18 October 2007 |
| | | | | US | 2010-0273221 | A1 | 28 October 2010 |
| | | | | US | 2012-0149072 | A1 | 14 June 2012 |
| | | | | US | 2015-0010962 | A1 | 08 January 2015 |
| | | | | US | 2015-0010963 | A1 | 08 January 2015 |
| | | | | US | 2015-0259716 | A1 | 17 September 2015 |
| | | | | US | 7790422 | B2 | 07 September 2010 |
| | | | | US | 8198053 | B2 | 12 June 2012 |
| | | | | US | 8859242 | B2 | 14 October 2014 |
| | | | | US | 9062335 | B2 | 23 June 2015 |
| | | | | US | 9334511 | B2 | 10 May 2016 |
| | | | | US | 9334512 | B2 | 10 May 2016 |
| | | | | WO | 2006-038695 | A1 | 13 April 2006 |
| KR | 10-2023-0029769 | A | 03 March 2023 | CN | 115867641 | A | 28 March 2023 |
| | | | | EP | 4176045 | A1 | 10 May 2023 |
| | | | | JP | 2023-536568 | A | 28 August 2023 |
| | | | | US | 2023-0313256 | A1 | 05 October 2023 |
| | | | | WO | 2022-003113 | A1 | 06 January 2022 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240116276
- KR 1020250101523
- US 8916213 B2
- US 9556463 B2
- KR 100159812
- KR 100397322